# FASCICULE DE BREVET EUROPEEN

(11) **EP 4 003 566 B1**
(45) Date de publication et mention de la délivrance du brevet: **18.06.2025**
(21) Numéro de dépôt: 20757382.5
(22) Date de dépôt: 24.07.2020
(51) Int. Cl.: B01D 15/18, B01D 15/24, B01D 15/36, B01D 15/38, A61L 2/18, C07K 1/18, C07K 1/22, A61L 2/00, C07K 1/36

(54) **PROCÉDÉ DE PURIFICATION D'UNE SUBSTANCE CIBLE AVEC INACTIVATION VIRALE**
VERFAHREN ZUR REINIGUNG EINER ZIELSUBSTANZ MIT VIRALER INAKTIVIERUNG
METHOD FOR PURIFYING A TARGET SUBSTANCE WITH VIRAL INACTIVATION

(30) Priorité: 26.07.2019 FR 1908574
(43) Date de publication de la demande: 01.06.2022
(73) Titulaire: Sartorius Chromatography Equipment, 54340 Pompey (FR)
(72) Inventeur: CHEVALIER, Jérôme, 57420 Pournoy La Grasse (FR); BEULAY, Jean-Luc, 67880 Krautergersheim (FR); FLOUQUET, Thomas, 54000 Nancy (FR); LAFONTAINE, Hélène, 54380 Saizerais (FR)
(74) Mandataire: Novagraaf International SA
(86) Numéro de dépôt international: PCT/FR2020/051360
(87) Numéro de publication internationale: WO 2021/019167

(56) Documents cités:
- EP-A1- 2 682 168
- WO-A1-2009/045897
- WO-A1-2011/017514
- WO-A1-2012/014183
- US-A1- 2014 255 994

## Description

### Domaine de l'invention

La présente invention concerne un procédé chromatographique de purification d'une substance cible comprenant une inactivation virale.

### Arrière-plan technique

Les anticorps et d'autres substances sont typiquement produits par culture cellulaire (cellules bactériennes ou eucaryotes). Les substances d'intérêt ainsi produites doivent ensuite être purifiées par diverses techniques afin d'éliminer les impuretés présentes dans le milieu de production. Pour cela, ces substances, et en particulier les anticorps, sont classiquement soumises à une étape de capture, par exemple par chromatographie d'affinité, et une ou plusieurs étapes de polissage (« *polishing steps* » en anglais), par exemple par chromatographie d'échange d'ions. Les procédés de purification comprennent également souvent une étape d'inactivation virale.

Par exemple, le document US 9,149,738 décrit un procédé de purification d'une molécule utilisant au moins trois unités de séparation connectées les unes aux autres en cercle et contenant une matrice d'affinité ou d'échange d'ions, dans lequel, de manière cyclique, une première colonne est chargée de l'échantillon à purifier en étant en communication fluidique avec la deuxième colonne tandis que dans le même temps la troisième colonne est éluée et régénérée. La molécule purifiée peut ensuite être soumise à une étape d'inactivation virale par une exposition à un pH acide en ligne ou en utilisant un réservoir tampon, puis à une étape de chromatographie « *en flux traversant* » (« *flow-through* » en anglais).

Le document WO 2012/078677 décrit un procédé continu de purification d'une biomolécule comprenant une étape de capture et une étape d'inactivation virale dans laquelle le flux de l'étape de capture est collecté dans un récipient où son pH est ajusté par une titration à l'acide. La solution est ensuite laissée le temps requis pour l'inactivation puis elle est transférée dans un autre récipient où son pH est réajusté en vue de son transfert à l'unité suivante, telle qu'une colonne de chromatographie d'échange de cations.

Les documents EP 2763771 et US 8,536,316 décrivent des procédés de purification dans lesquels l'inactivation virale est effectuée en passant un tampon d'inactivation virale sur une colonne après que celle-ci ait été chargée avec l'échantillon à traiter et avant l'élution de la molécule d'intérêt. Dans d'autres procédés, tel que décrits dans le document WO 2009/045897, l'inactivation virale est effectuée par l'élution de la molécule d'intérêt, retenue sur une première colonne de chromatographie, sous conditions acides et par le maintien de l'éluât acide dans une cuve un certain temps.

Le document EP 3130384 décrit un procédé de purification d'une molécule comprenant une étape de chromatographie en mode « *liaison* / *élution* » (« *bind and elute* » en anglais) et une étape de purification en mode « *flux traversant* » (charbon actif, chromatographie d'échange d'anions et chromatographie d'échange de cations). La chromatographie en mode « *liaison* / *élution* » peut être suivi d'une inactivation virale dans laquelle un agent d'inactivation virale est introduit en ligne dans une ligne de connexion comprenant un mélangeur statique, ou en utilisant un réservoir tampon. Un procédé assez semblable est décrit dans le document EP 2682168 A1.

Le document US 9,527,010 décrit un système pour la purification de biomolécules comprenant deux dispositifs de chromatographie connectés en série, un capteur couplé au conduit reliant les deux dispositifs de chromatographie mesurant une propriété telle que le pH, la conductivité ou la concentration d'une espèce, un dispositif permettant d'injecter un fluide au niveau du conduit reliant les deux dispositifs de chromatographie en fonction de la valeur de la propriété, ainsi qu'un filtre destiné à éliminer les virus en aval du deuxième dispositif de chromatographie.

Il existe un besoin de fournir un procédé de purification efficace comprenant une inactivation virale, pouvant être mis en œuvre dans une seule installation et pouvant être contrôlé par un seul programme d'ordinateur, permettant ainsi une installation avec un encombrement plus réduit, une purification plus rapide et de plus grande qualité.

### Résumé de l'invention

L'invention concerne en premier lieu un procédé de purification d'une substance cible à partir d'un fluide à traiter comprenant au moins une impureté, dans une installation comprenant :
- une première unité de séparation comprenant au moins une colonne de chromatographie, une entrée de fluide et une sortie de fluide ;
- une deuxième unité de séparation comprenant au moins une colonne de chromatographie, une entrée de fluide, une sortie de fluide et une ligne de contournement contournant l'au moins une colonne de chromatographie de la deuxième unité de séparation;
- une première cuve en connexion fluidique avec une sortie de fluide de la première unité de séparation et avec une entrée de fluide de la deuxième unité de séparation ; et
- une seconde cuve en connexion fluidique avec au moins une entrée de fluide et une sortie de fluide de la deuxième unité de séparation ;
le procédé comprenant les étapes suivantes :
- la fourniture d'un flux du fluide à traiter ;
- le traitement du flux de fluide à traiter par une étape de chromatographie dans la première unité de séparation;
- la collecte d'une fraction enrichie en la substance cible dans la première cuve ;
- l'inactivation virale de la fraction enrichie en la substance cible, ladite inactivation virale comprenant :
   ▪ le passage de la fraction enrichie en la substance cible collectée dans la première cuve dans la deuxième unité de séparation, par la ligne de contournement;
   ▪ le passage d'une solution d'inactivation virale dans la deuxième unité de séparation, par la ligne de contournement ;
   ▪ le mélange de la fraction enrichie en la substance cible et de la solution d'inactivation virale ; et
   ▪ la collecte dans la seconde cuve du mélange de la fraction enrichie en la substance cible et de la solution d'inactivation virale ;
   de manière à obtenir une fraction déplétée en virus actif ;
- le traitement de la fraction déplétée en virus actif par une étape de chromatographie dans la deuxième unité de séparation; et
- la collecte en sortie de la deuxième unité de séparation d'une fraction plus enrichie en la substance cible.

Dans des modes de réalisation, le traitement du flux de fluide à traiter par une étape de chromatographie dans la première unité de séparation est un traitement par chromatographie d'affinité.

Dans des modes de réalisation, le traitement de la fraction déplétée en virus actif par une étape de chromatographie dans la deuxième unité de séparation est un traitement par chromatographie d'échange d'ions, de préférence un traitement par chromatographie d'échange d'anions.

Dans des modes de réalisation, l'étape de passage de la fraction enrichie en la substance cible collectée dans la première cuve dans la deuxième unité de séparation, par la ligne de contournement, et l'étape de passage d'une solution d'inactivation virale dans la deuxième unité de séparation, par la ligne de contournement, sont concomitantes, l'étape de mélange de la fraction enrichie en la substance cible et de la solution d'inactivation virale étant effectuée en ligne dans la ligne de contournement de la deuxième unité de séparation.

Dans des modes de réalisation, l'étape de passage de la fraction enrichie en la substance cible collectée dans la première cuve dans la deuxième unité de séparation, par la ligne de contournement, et l'étape de passage d'une solution d'inactivation virale dans la deuxième unité de séparation, par la ligne de contournement, sont successives, l'étape de mélange de la fraction enrichie en la substance cible et de la solution d'inactivation virale étant effectuée dans la seconde cuve.

Dans des modes de réalisation, le mélange de la fraction enrichie en la substance cible et de la solution d'inactivation virale collecté dans la seconde cuve est stocké dans la seconde cuve pendant au moins 15 min, de préférence pendant une durée allant de 20 à 45 min, de préférence encore sous agitation.

Dans des modes de réalisation, l'inactivation virale de la fraction enrichie en la substance cible comprend en outre :
a) le passage du mélange de la fraction enrichie en la substance cible et de la solution d'inactivation virale collecté dans la seconde cuve dans la deuxième unité de séparation, par la ligne de contournement;
b) la collecte du mélange en sortie de la deuxième unité de séparation dans la seconde cuve;
   les étapes a) et b) étant optionnellement répétées une ou plusieurs fois ou effectuées en continu ; et
c) optionnellement le stockage du mélange dans la seconde cuve, de préférence sous agitation ;
l'ensemble des étapes a), b), éventuellement répétées, ou éventuellement effectuées en continu, et de l'étape optionnelle c) étant de préférence effectuées pendant une durée d'au moins 15 min, plus préférentiellement pendant une durée de 20 à 45 min.

Dans des modes de réalisation, la deuxième unité de séparation comprend en outre un détecteur de pH et/ou un détecteur de température, de préférence un détecteur en ligne, l'inactivation virale de la fraction enrichie en la substance cible comprenant en outre la mesure et, éventuellement le réajustement, du pH et/ou de la température du mélange de la fraction enrichie en la substance cible et de la solution d'inactivation virale passant dans la deuxième unité de séparation.

Dans des modes de réalisation, la solution d'inactivation virale est une solution acide.

Dans des modes de réalisation, le mélange de la fraction enrichie en la substance cible et de la solution acide a un pH inférieur ou égal à 5, de préférence inférieure ou égal à 4.

Dans des modes de réalisation, le procédé comprend, avant l'étape de traitement de la fraction déplétée en virus actif par une étape de chromatographie dans la deuxième unité de séparation, une étape d'ajout d'une solution basique dans la fraction déplétée en virus actif.

Dans des modes de réalisation, l'ajout de la solution basique dans la fraction déplétée en virus actif est effectué en ligne dans la deuxième unité de séparation.

Dans des modes de réalisation, l'ajout de la solution basique dans la fraction déplétée en virus actif est effectué dans la seconde cuve, en passant de préférence la solution basique dans la deuxième unité de séparation, par la ligne de contournement.

Dans des modes de réalisation, le procédé comprend au moins une étape de rinçage de la deuxième unité de séparation dans laquelle un tampon de rinçage est passé dans la deuxième unité de séparation, par la ligne de contournement, de préférence après l'étape d'inactivation virale de la fraction enrichie en la substance cible et/ou avant l'étape de traitement de la fraction déplétée en virus actif par une étape de chromatographie dans la deuxième unité de séparation et/ou après l'étape de traitement de la fraction déplétée en virus actif par une étape de chromatographie dans la deuxième unité de séparation.

Dans des modes de réalisation, l'installation comprend en outre une troisième unité de séparation comprenant au moins une colonne de chromatographie, une entrée de fluide et une sortie de fluide, dans laquelle une entrée de fluide est en connexion fluidique avec une sortie de fluide de la deuxième unité de séparation ;
le procédé comprenant en outre les étapes suivantes :
- le traitement de la fraction plus enrichie en la substance cible par une étape de chromatographie dans la troisième unité de séparation ; et
- la collecte en sortie de la troisième unité de séparation d'une fraction purifiée de la substance cible.

Dans des modes de réalisation, le traitement de la fraction plus enrichie en la substance cible par une étape de chromatographie dans la troisième unité de séparation est un traitement par chromatographie d'échange d'ions, de préférence un traitement par chromatographie d'échange de cations.

Dans des modes de réalisation, le fluide à traiter est un surnageant de culture cellulaire.

Dans des modes de réalisation, la substance cible est un anticorps.

Dans des modes de réalisation, la première unité de séparation est une unité multicolonne.

L'invention concerne également un programme d'ordinateur comprenant des instructions de code de programme pour l'exécution des étapes du procédé tel que décrit ci-dessus lorsque ledit programme est exécuté sur un ordinateur.

L'invention concerne également un support de stockage lisible par ordinateur sur lequel est enregistré un programme d'ordinateur tel que décrit ci-dessus.

L'invention concerne également un système comprenant un processeur couplé à une mémoire sur laquelle est enregistré un programme d'ordinateur tel que décrit ci-dessus.

La présente invention permet de répondre au besoin exprimé ci-dessus. Elle fournit plus particulièrement un procédé de purification d'une substance cible avec inactivation virale permettant l'utilisation d'un système de purification moins encombrant, ainsi qu'une purification plus rapide et plus efficace.

Cela est accompli grâce à la mise en œuvre de l'étape d'inactivation virale au moyen de la deuxième unité de séparation, par une ligne de contournement des dispositifs de séparation (tels que les colonnes). Cette ligne de contournement a l'avantage d'opérer des flux, mélangeant par exemple le produit contenant la substance cible avec une solution d'inactivation virale, plus importants qu'une unité de chromatographie standard. Cela permet d'éviter l'utilisation d'une autre installation différente pour effectuer l'inactivation virale. Ainsi, le procédé de purification peut être mis en œuvre entièrement dans une seule installation, commandée par un unique logiciel, ce qui permet un gain de place, de temps et une meilleure qualité de la purification.

En outre, comparé à un procédé dans lequel l'inactivation virale est effectuée sur une colonne, en particulier de chromatographie d'affinité, sur laquelle a été chargé le produit à traiter, par l'élution de la molécule d'intérêt avec une solution d'inactivation virale, telle qu'une solution acide, le procédé selon l'invention permet d'éviter le passage de la solution d'inactivation virale sur la colonne de chromatographie. Le passage de la solution d'inactivation virale sur une colonne de chromatographie est désavantageux puisqu'il est ensuite nécessaire de soigneusement rincer la colonne.

En outre, les volumes de produit traités par la première unité de séparation (par exemple une unité de chromatographie d'affinité) sont généralement plus importants que les volumes traités par l'unité de séparation suivante (par exemple une unité de chromatographie d'échange d'ions). Cela est dû au fait que, lors de la séparation dans la première unité de séparation, une partie des impuretés contenues dans le produit initial à traiter sont éliminées, ce qui réduit le volume de la fraction collectée en sortie de cette première unité de séparation par rapport au volume de produit initial. Lorsque l'inactivation virale est effectuée sur les colonnes de chromatographie d'affinité de la première unité de séparation (par passage de la solution d'inactivation virale directement sur les colonnes), lesdites colonnes de chromatographie d'affinité ne sont plus disponibles pour effectuer la séparation pendant toute la durée de l'inactivation virale. Le procédé selon l'invention permet un gain de temps par rapport à un tel procédé puisque la première unité de séparation peut être utilisée sans immobilisation due à une inactivation virale, l'étape d'inactivation virale étant réalisée pendant le temps mort de la deuxième unité de séparation dû au plus faible volume de produit traité dans la deuxième unité de séparation.

### Brève description des figures

La **figure 1** représente une étape du procédé de purification selon un premier, un deuxième et un troisième mode de réalisation.
La **figure 2** représente une autre étape du procédé de purification selon un premier et un deuxième mode de réalisation.
La **figure 3** représente une autre étape du procédé de purification selon un premier mode de réalisation.
La **figure 4** représente une autre étape du procédé de purification selon un premier mode de réalisation.
La **figure 5** représente une autre étape du procédé de purification selon un premier mode de réalisation.
La **figure 6** représente une autre étape du procédé de purification selon un premier mode de réalisation.
La **figure 7** représente une autre étape du procédé de purification selon un premier mode de réalisation.
La **figure 8** représente une autre étape du procédé de purification selon un deuxième mode de réalisation.
La **figure 9** représente une autre étape du procédé de purification selon un deuxième mode de réalisation.
La **figure 10** représente une autre étape du procédé de purification selon un deuxième mode de réalisation.
La **figure 11** représente une autre étape du procédé de purification selon un deuxième mode de réalisation.
La **figure 12** représente une autre étape du procédé de purification selon un deuxième mode de réalisation.
La **figure 13** représente une autre étape du procédé de purification selon un deuxième mode de réalisation.
La **figure 14** représente une autre étape du procédé de purification selon un deuxième mode de réalisation.
La **figure 15** représente une autre étape du procédé de purification selon un troisième mode de réalisation.
La **figure 16** représente une autre étape du procédé de purification selon un troisième mode de réalisation.
La **figure 17** représente une autre étape du procédé de purification selon un troisième mode de réalisation.
La **figure 18** représente une autre étape du procédé de purification selon un troisième mode de réalisation.
La **figure 19** représente une autre étape du procédé de purification selon un troisième mode de réalisation.
La **figure 20** représente une autre étape du procédé de purification selon un troisième mode de réalisation.
La **figure 21** représente une autre étape du procédé de purification selon un troisième mode de réalisation.
La **figure 22** représente une autre étape du procédé de purification selon un troisième mode de réalisation.
La **figure 23** représente une étape du procédé de purification selon un premier, un deuxième et un troisième mode de réalisation utilisant une seule cuve.
La **figure 24** représente une autre étape du procédé de purification selon un premier et un deuxième mode de réalisation utilisant une seule cuve.
La **figure 25** représente une autre étape du procédé de purification selon un premier et deuxième mode de réalisation utilisant une seule cuve.

### Description détaillée

L'invention est maintenant décrite plus en détail et de façon non limitative dans la description qui suit.

L'invention concerne un procédé de purification d'une substance cible à partir d'un fluide à traiter.

### Fluide à traiter

La substance cible peut être notamment toute molécule ou macromolécule mais est de préférence une molécule ou macromolécule biologique. De manière plus préférée, la substance cible est une protéine ou une glycoprotéine. En particulier, la substance cible peut être une protéine recombinante, telle qu'un anticorps ou encore un vecteur viral.

Au sens de la présente invention, le terme « *anticorps* » inclut les anticorps complets et les fragments ou dérivés de ceux-ci, ainsi que les protéines fusions comprenant un fragment d'anticorps (tel qu'un fragment Fc), du moment qu'ils contiennent un domaine de liaison spécifique à un ligand. Les anticorps peuvent être monoclonaux ou polyclonaux, monomériques ou polymériques, monospécifiques ou multispécifiques (par exemple bispécifiques).

Comme exemple d'anticorps, on peut citer les anticorps humains, et en particulier les IgG (telles que les IgG1, IgG2, IgG3 et IgG4), les IgM, les IgA (telles que les IgA1 et/ou IgA2), les IgD et les IgE, les anticorps canins, et en particulier les IgGA, les IgGB, les IgGC et les IgGD, les anticorps de poulet, en particulier les IgA, les IgD, les IgE, les IgG, les IgM et les IgY, les anticorps de chèvre, en particulier les IgG, les anticorps de souris, en particulier les IgG, les IgD, les IgE et les IgM, les anticorps porcins, en particulier les IgG, les IgD, les IgE et les IgM, les anticorps de rat, en particulier les IgG, les IgD, les IgE et les IgM, les anticorps chimériques, les anticorps humanisés, ainsi que les fragments ou dérivés de ceux-ci.

Comme exemples de fragments, on peut citer les fragments Fab, Fab', F(ab')₂, Fc et Fv.

La substance à traiter peut aussi être un mélange des substances mentionnées ci-dessus.

Le fluide à traiter contient la substance cible à purifier et une ou plusieurs impuretés. De préférence, le fluide à traiter est un surnageant de culture cellulaire. Alternativement, le fluide à traiter peut être un fluide de culture cellulaire, comprenant par exemple des cellules et/ou des débris cellulaires insolubles, ou un bouillon d'enzymation.

Les impuretés présentes dans le fluide à traiter peuvent comprendre des protéines de cellules hôtes (ou HCP pour « *host cell proteins* »), de l'ADN et/ou fragment d'ADN, de l'ARN et/ou fragment d'ARN, des microorganismes, des virus, des endotoxines, des lipides, des composants du milieu de culture cellulaire ou un mélange de ceux-ci.

Le fluide à traiter comprend de préférence des virus. Le procédé de l'invention permet d'inactiver ceux-ci.

Le fluide à traiter peut avoir subi une clarification en tant que prétraitement. L'étape de clarification consiste à réduire ou éliminer les matières en suspension. Elle peut être réalisée par exemple au moyen d'une filtration à l'aide d'un filtre à poche ou bobine, filtre-panier ou filtre-presse, une centrifugation, une décantation, une filtration sur terre avec ou sans pré-couche et/ou nourrissage, une filtration frontale ou encore une filtration tangentielle sur membrane de type microfiltration ou ultrafiltration, ou encore une filtration sur diatomées ou sur membrane céramique, ou une filtration sur charbon actif ou encore une précipitation.

### Installation et procédé de purification général

Le procédé selon l'invention est mis en œuvre dans une installation comprenant :
- une première unité de séparation comprenant au moins une entrée de fluide et au moins une sortie de fluide ;
- une deuxième unité de séparation comprenant au moins une entrée de fluide et au moins une sortie de fluide ;
- une première cuve; et
- une seconde cuve.

La première unité de séparation peut être par exemple une unité de chromatographie, incluant des colonnes et/ou membranes de chromatographie, mais aussi une unité de filtration, de centrifugation, ou de précipitation. La première unité de séparation comprend au moins un dispositif de séparation.

De préférence, la première unité de séparation est une unité de chromatographie et elle comprend une ou plusieurs colonnes et/ou membranes de chromatographie en tant que dispositifs de séparation. Lorsque plusieurs colonnes et/ou membranes sont présentes, elles peuvent être toutes connectées en série, toutes connectées en parallèle, ou l'unité de séparation peut comprendre certaines colonnes et/ou membranes connectées en série et certaines colonnes et/ou membranes connectées en parallèle. De préférence, la première unité de séparation est une unité multicolonne, c'est-à-dire qu'elle comprend au moins deux colonnes.

La ou les colonnes et/ou membranes de chromatographie de la première unité de séparation contiennent une phase stationnaire. De préférence, toutes les colonnes et/ou membranes de la première unité de séparation ont une phase stationnaire identique. Alternativement, les colonnes et/ou membranes peuvent avoir des phases stationnaires différentes.

La phase stationnaire est de préférence une résine de chromatographie d'affinité. Des résines de chromatographie appropriées pour la première unité de séparation sont des résines ayant des ligands Protéine A, Protéine G, Protéine L et/ou des variants fonctionnels de ceux-ci.

La phase stationnaire des colonnes et/ou membranes de la première unité de séparation peut aussi être une résine échangeuse d'ions, telle qu'une résine cationique, une résine cationique en mode mixte, une résine anionique, une résine anionique en mode mixte, forte ou faible, ou un mélange de celles-ci, ou une résine de chromatographie d'interaction hydrophobe ou encore une résine d'exclusion de taille.

L'unité de filtration peut comprendre un ou plusieurs filtres stériles, filtres charbon, filtres à membrane à circulation tangentielle ou frontale, et/ou filtres de rétention de virus en tant que dispositifs de séparation.

Une entrée de fluide de la première unité de séparation est de préférence en connexion fluidique avec un réservoir contenant le fluide à traiter. D'autres réservoirs peuvent également être en connexion fluidique avec une entrée de fluide de la première unité de séparation, par exemple un réservoir contenant un éluant. Des pompes peuvent être présentes pour amener les différents fluides dans la première unité de séparation.

Par « *connexion fluidique* » entre deux dispositifs ou parties de dispositif, on entend que lesdits dispositifs ou parties de dispositif sont connectés entre eux par une ligne de connexion permettant à un fluide de s'écouler d'un dispositif à l'autre. Cette ligne de connexion peut être directe ou bien être interrompue par un ou plusieurs éléments tels que des vannes, des unités de séparations ou tout autre élément de l'installation. Une connexion fluidique peut être permanente, c'est-à-dire qu'un fluide s'écoule de manière permanente dans la ligne de connexion, ou non permanente, c'est-à-dire que l'écoulement dans la ligne de connexion peut être stoppé et repris, par exemple au moyen d'une vanne présente dans la ligne de connexion. Lorsqu'un fluide s'écoule effectivement d'un dispositif à un autre (ou d'une partie de dispositif à une autre), les deux dispositifs (ou parties de dispositif) sont en « *communication fluidique* ».

Un flux du fluide à traiter est soumis à une étape de séparation, de préférence une étape de chromatographie, dans la première unité de séparation. Plus préférentiellement, il s'agit d'une chromatographie d'affinité. Alternativement, il peut s'agir d'une chromatographie d'échanges d'ions, telle qu'une chromatographie d'échanges d'anions ou d'échange de cations, d'une chromatographie d'interaction hydrophobe, ou d'une chromatographie d'exclusion de taille. La mise en œuvre de telles étapes de chromatographie est bien connue de l'homme du métier.

Alternativement, l'étape de séparation est une étape de filtration, de centrifugation ou de précipitation.

En sortie de cette étape de séparation (par exemple de l'étape de chromatographie), on obtient une fraction enrichie en la substance cible et une fraction enrichie en impuretés.

Par « *fraction enrichie en la substance cible* », on entend une fraction dans laquelle le rapport des concentrations molaires substance cible / impuretés totales est supérieur à celui du flux en entrée de la séparation (par exemple en entrée de chromatographie). De manière similaire, une « *fraction enrichie en impuretés* » désigne une fraction dans laquelle le rapport des concentrations molaires substance cible / impuretés totales est inférieur à celui du flux en entrée de la séparation (par exemple en entrée de chromatographie).

Une sortie de fluide de la première unité de séparation est en connexion fluidique avec la première cuve et la fraction enrichie en la substance cible est collectée dans la première cuve.

La première cuve est en connexion fluidique avec une entrée de fluide de la deuxième unité de séparation.

La première cuve a de préférence une capacité de stockage égale ou supérieur au volume reçu de la première unité de séparation d'un cycle entier de production. Le volume d'un cycle entier de production correspond au volume de fraction enrichie en la substance cible issu de la première unité de séparation. Cette capacité de stockage peut être divisée en plusieurs premières cuves.

La deuxième unité de séparation peut être par exemple une unité de chromatographie, incluant des colonnes et/ou des membranes de chromatographie, mais aussi une unité de filtration, de centrifugation, ou de précipitation. La deuxième unité de séparation comprend au moins un dispositif de séparation.

De préférence, la deuxième unité de séparation est une unité de chromatographie et elle comprend au moins une colonne ou membrane de chromatographie en tant que dispositifs de séparation. Lorsque plusieurs colonnes et/ou membranes sont présentes, elles peuvent être toutes connectées en série, toutes connectées en parallèle, ou l'unité de séparation peut comprendre certaines colonnes et/ou membranes connectées en série et certaines colonnes et/ou membranes connectées en parallèle.

La ou les colonnes et/ou membranes de chromatographie de la deuxième unité de séparation contiennent une phase stationnaire. De préférence, toutes les colonnes et/ou membranes de la deuxième unité de séparation ont une phase stationnaire identique. Alternativement, les colonnes et/ou membranes peuvent avoir des phases stationnaires différentes.

De manière préférée, la deuxième unité de séparation comprend une seule colonne ou membrane de chromatographie.

La phase stationnaire est de préférence une résine échangeuse d'ions. De manière plus préférentielle, il s'agit d'une résine échangeuse d'anions, ou d'anions en mode mixte. Alternativement, il peut s'agir d'une résine échangeuse de cations ou de cations en mode mixte. Dans d'autres modes de réalisation, la phase stationnaire peut être du charbon actif.

L'unité de filtration peut comprendre un ou plusieurs filtres stériles, filtres charbon, filtres à membrane à circulation tangentielle ou frontale, et/ou filtres de rétention de virus en tant que dispositifs de séparation.

La deuxième unité de séparation comprend également une ligne de contournement. Cette ligne de contournement permet de contourner (ou court-circuiter) le dispositif de séparation, par exemple la ou les colonnes et/ou membranes de chromatographie, de la deuxième unité. Lorsque la deuxième unité de séparation comprend plusieurs colonnes et/ou membranes, la ligne de contournement contourne toutes lesdites colonnes et/ou membranes de la deuxième unité de séparation. En d'autres termes, lorsqu'un fluide traverse la deuxième unité de séparation en passant par cette ligne de contournement, le fluide ne passe pas dans le dispositif de séparation (par exemple la ou les colonnes et/ou membranes de chromatographie) de la deuxième unité de séparation. La ligne de contournement est reliée à au moins une entrée de fluide de la deuxième unité de séparation à l'une de ses extrémités et à au moins une sortie de fluide de la deuxième unité de séparation à l'autre de ses extrémités.

Une sortie de la deuxième unité de séparation est en connexion fluidique avec la seconde cuve. Au moins une entrée de fluide de la deuxième unité de séparation est également en connexion fluidique avec la seconde cuve.

La seconde cuve a une capacité de stockage égale ou supérieure au volume de la (ou des) première(s) cuve(s). Cette capacité de stockage peut être divisée en plusieurs secondes cuves.

De préférence, la ou les entrées de fluide de la deuxième unité de séparation sont en connexion fluidique avec un ou plusieurs réservoirs, par exemple un réservoir de solution d'inactivation virale (par exemple une solution acide), un réservoir de solution basique, un ou plusieurs réservoirs de tampon de rinçage et/ou un réservoir d'éluant ; de préférence, chaque réservoir est en connexion fluidique avec une entrée de fluide différente de la deuxième unité de séparation. Les fluides contenus dans ces réservoirs, ou dans les première et seconde cuves peuvent être apportés à la deuxième unité de séparation par une ou plusieurs pompes.

La fraction collectée dans la première cuve est ensuite soumise à une étape d'inactivation virale.

Cette étape d'inactivation virale comprend :
▪ le passage de la fraction enrichie en la substance cible collectée dans la première cuve dans la deuxième unité de séparation, par la ligne de contournement.
▪ le passage d'une solution d'inactivation virale dans la deuxième unité de séparation, par la ligne de contournement ;
▪ le mélange de la fraction enrichie en la substance cible et de la solution d'inactivation virale ; et
▪ la collecte dans la seconde cuve du mélange de la fraction enrichie en la substance cible et de la solution d'inactivation virale.

Lors de cette étape, ni la fraction enrichie en la substance cible, ni la solution d'inactivation virale ne traverse le dispositif de séparation (par exemple la ou les colonnes de chromatographie) de la deuxième unité de séparation.

De manière avantageuse, la solution d'inactivation virale est une solution acide. Alternativement, ou en addition, la solution d'inactivation peut comprendre un autre agent d'inactivation virale, par exemple un détergent tel que le Triton X-100.

Les passages, dans la deuxième unité de séparation, de la fraction enrichie en la substance cible et de la solution d'inactivation virale peuvent être simultanés ou successifs.

Lorsque ces passages sont simultanés (ou concomitants), le mélange de la fraction enrichie en la substance cible et de la solution d'inactivation virale a lieu en ligne dans la deuxième unité de séparation, et notamment dans la ligne de contournement. En sortie de la deuxième unité de séparation, le mélange est ensuite collecté dans la seconde cuve.

Par « *mélange effectué en ligne* », on entend au sens de la présente invention que le mélange est effectué dans une conduite ou ligne de l'unité de séparation. Un mélange effectué en ligne n'est pas réalisé dans un réservoir ou dans une cuve.

Lorsque ces passages sont successifs, de préférence le passage de la solution d'inactivation virale est effectué après le passage de la fraction enrichie en la substance cible. La fraction enrichie en la substance cible et la solution d'inactivation virale sont alors chacune, en sortie de la deuxième unité de séparation, collectées dans la seconde cuve. Le mélange de la fraction enrichie en la substance cible et de la solution d'inactivation virale est réalisé dans la seconde cuve.

L'ajout de la solution d'inactivation virale dans la fraction enrichie en la substance cible a pour but d'inactiver la totalité ou une partie des virus susceptibles d'être présents. Par « *inactiver un virus* », on entend supprimer l'activité biologique de ce virus et en particulier son pouvoir infectieux. Au contraire, un « *virus actif* » est un virus conservant son activité biologique et notamment son pouvoir infectieux. Lorsque la solution d'inactivation virale est une solution acide, l'ajout de solution acide dans la fraction enrichie en la substance cible a pour but d'ajuster le pH du mélange à un pH suffisamment bas pour inactiver la totalité ou une partie des virus susceptibles d'être présents. De préférence, le pH du mélange de la fraction enrichie en la substance cible et de la solution acide est inférieur ou égal à 5, plus préférentiellement inférieur ou égal à 4, encore plus préférentiellement de 3 à 3,8, par exemple de 3 à 3,2, ou de 3,2 à 3,4, ou de 3,4 à 3,6, ou de 3,6 à 3,8, ou de 3,8 à 4, ou de 4 à 4,5 ou de 4,5 à 5.

La solution acide a de préférence un pH compris entre 3 et 5. Des exemples de solutions acides utilisables dans le procédé selon l'invention sont une solution d'acide acétique et/ou d'acide caprylique.

La fraction enrichie en la substance cible doit rester en contact avec la solution d'inactivation virale pendant une certaine durée pour que l'inactivation virale soit effective. Par exemple, lorsque la solution d'inactivation virale est une solution acide, le mélange de la fraction enrichie en la substance cible et de la solution acide doit être maintenu au pH tel que défini ci-dessus pendant une certaine durée pour que l'inactivation virale soit effective. De préférence, cette durée vaut au moins 5 minutes, de manière plus préférée au moins 15 minutes, plus préférentiellement de 20 à 45 minutes, encore plus préférentiellement de 25 à 35 minutes. Selon des modes de réalisation, la durée vaut de 5 à 10 min, ou de 10 à 15 min, ou de 15 à 20 min, ou de 20 à 25 min, ou de 25 à 30 min, ou de 30 à 35 min, ou de 35 à 40 min, ou de 40 à 45 min.

Dans des modes de réalisation, le mélange de la fraction enrichie en la substance cible et de la solution d'inactivation virale collecté dans la seconde cuve est stocké dans la seconde cuve pendant la durée mentionnée ci-dessus.

Dans des modes de réalisations, le mélange de la fraction enrichie en la substance cible et de la solution d'inactivation virale collecté dans la seconde cuve subit les étapes suivantes :
a) le mélange de la fraction enrichie en la substance cible et de la solution d'inactivation virale passe dans la deuxième unité de séparation, par la ligne de contournement ;
b) le mélange en sortie de la deuxième unité de séparation est collecté dans la seconde cuve ;
   les étapes a) et b) peuvent optionnellement être répétées une ou plusieurs fois, ou être mises en œuvre en continu pendant une certaine durée ; et
c) optionnellement le mélange est stocké dans la seconde cuve.

La séquence des étapes a), b), de l'éventuelle répétition des étapes a) et b), de l'éventuelle mise en œuvre en continu des étapes a) et b), et de l'étape optionnelle c) a de préférence une durée telle que mentionnée ci-dessus.

De manière avantageuse, la seconde cuve comprend des moyens d'agitation, tels que des agitateurs mécaniques rotatifs (par exemple lorsque la cuve est réutilisable) ou encore des agitateurs à entraînement magnétique (par exemple lorsque la cuve est à usage unique). De manière préférée, le stockage du mélange dans la seconde cuve est effectué sous agitation.

La deuxième unité de séparation peut comprendre au moins un détecteur.

Avantageusement, le détecteur est un détecteur en ligne. Par « *détecteur en ligne* », on entend au sens de la présente invention un détecteur positionné sur une conduite ou ligne de l'unité de séparation. Par « *détecteur en ligne* », on entend également un détecteur en dérivation dont le prélèvement est positionné sur une conduite ou ligne de l'unité de séparation.

De préférence, le détecteur est un détecteur de pH, un détecteur de température, un détecteur de conductimétrie, un densimètre, un polarimètre, un réfractomètre, un spectromètre infrarouge, proche infrarouge, Raman ou UV/visible ou un appareil de résonance magnétique nucléaire en ligne. Plusieurs détecteurs peuvent être présents, de préférence choisis parmi les détecteurs cités ci-dessus.

De manière avantageuse, la deuxième unité de séparation comprend un détecteur de pH et/ou un détecteur de température.

Ces détecteurs peuvent permettre de mesurer une grandeur, telle que le pH, la température, la conductivité, la densité, le pouvoir rotatoire, l'indice de réfraction, l'absorbance ou l'émission d'un rayonnement spectroscopique ou la résonance magnétique nucléaire des fluides traversant la deuxième unité de séparation, et en particulier du mélange de la fraction enrichie en la substance cible et de la solution d'inactivation virale.

De manière particulièrement avantageuse, une mesure du pH et/ou de la température et/ou d'une autre grandeur telle que celles mentionnées ci-dessus, est effectuée sur le mélange de la fraction enrichie en la substance cible et de la solution d'inactivation virale passant dans la deuxième unité de séparation. De manière encore plus préférée, cette mesure est effectuée à chaque passage du mélange de la fraction enrichie en la substance cible et de la solution d'inactivation virale dans la deuxième unité de séparation ou en continu lorsque ledit mélange passe en continu dans la deuxième unité de séparation. Cela est particulièrement avantageux lorsque les étapes a) et b) ci-dessus sont mises en œuvre de manière continue ou qu'elles sont répétées une ou plusieurs fois. Ainsi, si la valeur de la grandeur mesurée (telle que le pH et/ou la température) dévie de la valeur souhaitée de ladite grandeur, cette dernière peut être réajustée. Par exemple, le pH peut être réajusté en ajoutant une solution acide (qui peut être la même que la solution d'inactivation virale) ou une solution basique dans la deuxième unité de séparation, par la ligne de contournement, de manière à ce qu'elle se mélange avec le mélange de la fraction enrichi en la substance cible et de la solution d'inactivation virale.

De manière alternative ou de manière additionnelle, la deuxième cuve peut comprendre un détecteur. La nature du détecteur peut être telle que décrite ci-dessus. Ces détecteurs peuvent permettre de mesurer une grandeur, telle que le pH, la température, la conductivité, la densité, le pouvoir rotatoire, l'indice de réfraction, l'absorbance ou l'émission d'un rayonnement spectroscopique ou la résonance magnétique nucléaire des fluides contenus dans la deuxième cuve, et en particulier du mélange de la fraction enrichie en la substance cible et de la solution d'inactivation virale.

De manière avantageuse, la deuxième cuve comprend un détecteur de pH et/ou un détecteur de température.

Une mesure du pH et/ou de la température et/ou d'une autre grandeur telle que celles mentionnées ci-dessus, peut être effectuée sur le mélange de la fraction enrichie en la substance cible et de la solution d'inactivation virale contenus dans la deuxième cuve. Cela est particulièrement avantageux lorsque ledit mélange est réalisé et/ou stocké dans la seconde cuve. Ainsi, si la valeur de la grandeur mesurée (telle que le pH et/ou la température) dévie de la valeur souhaitée de ladite grandeur, cette dernière peut être réajustée. Par exemple, le pH du mélange peut être réajusté en injectant une solution acide (qui peut être la même que la solution d'inactivation virale) ou une solution basique dans la seconde cuve en passant dans la deuxième unité de séparation, par la ligne de contournement.

Suite à l'étape d'inactivation virale, on obtient une fraction déplétée en virus actif. Par « *fraction déplétée en virus actif* », on entend une fraction dans laquelle le rapport des concentrations molaires virus actifs / virus totaux est inférieur à celui de la fraction avant l'étape d'inactivation virale. De préférence, la fraction déplétée en virus actif est essentiellement dépourvue de virus actifs.

De manière avantageuse, un rinçage de la deuxième unité de séparation est effectué après l'étape d'inactivation virale en faisant passer un tampon de rinçage dans la deuxième unité de séparation, par la ligne de contournement. Pendant cette étape de rinçage, la fraction déplétée en virus actif est stockée dans la seconde cuve. De manière préférentielle, la deuxième unité de séparation n'est pas en communication fluidique avec la seconde cuve pendant cette étape de rinçage et le tampon de rinçage, après passage dans la ligne de contournement, est de préférence éliminé de l'installation en étant orienté vers une ligne de collecte des déchets. Toutefois, la deuxième unité de séparation peut être en communication fluidique avec la seconde cuve pendant le rinçage, de préférence pendant une partie seulement du rinçage, plus préférentiellement pendant une partie au début du rinçage. Cela permet de récupérer dans la seconde cuve le mélange de fraction enrichie en la substance cible et de solution d'inactivation virale resté dans la deuxième unité de séparation. L'orientation du tampon de rinçage, après son passage dans la ligne de contournement, vers une ligne de collecte des déchets ou vers la seconde cuve peut être décidée sur l'atteinte d'une valeur de consigne de pH, ou de conductivité, ou d'un volume passé de tampon de rinçage ou encore sur une temporisation.

L'ensemble des rinçages décrits dans la présente demande peuvent être effectués au moyen d'un tampon de rinçage qui peut être de l'eau purifiée ou de l'eau pour préparation injectable, additionnée ou non de sels. En outre, pour l'ensemble des rinçages décrits dans la présente demande, une grandeur du tampon de rinçage, par exemple son pH, peut être mesurée par un détecteur placé dans la deuxième unité de séparation, de préférence un détecteur en ligne. Le débit et/ou volume d'entrée du tampon de rinçage lors de ces rinçages peut alors être modifié en fonction de la grandeur mesurée (par exemple en fonction du pH mesuré).

De préférence, une solution basique est ajoutée à la fraction déplétée en virus actif, en particulier dans les modes de réalisation dans lesquels la solution d'inactivation virale est une solution acide. Cela permet de réajuster le pH de cette fraction à un pH adapté aux opérations subséquentes.

La solution basique a de préférence un pH compris entre 7 et 9. Des exemples de solutions basiques utilisables dans le procédé selon l'invention sont une solution de soude et/ou de phosphate monosodique (NaH₂PO₄).

Dans des modes de réalisation, la solution basique est ajoutée à la fraction déplétée en virus actif en ligne dans la deuxième unité de séparation. Par « *ajout en ligne* », on entend au sens de la présente invention que l'ajout est effectué au niveau d'une conduite ou ligne de l'unité de séparation. Un ajout effectué en ligne n'est pas réalisé dans un réservoir ou dans une cuve. Dans ces modes de réalisation, la solution basique et la fraction déplétée en virus actif (contenue dans la seconde cuve) sont passées dans la deuxième unité de séparation simultanément, de sorte qu'elles se mélangent dans la deuxième unité de séparation.

Dans d'autres modes de réalisation, la solution basique est ajoutée dans la fraction déplétée en virus actif dans la seconde cuve. De préférence, dans ces modes de réalisation, la solution basique est ajoutée à la fraction déplétée en virus actif en passant dans la seconde unité de séparation, par la ligne de contournement, la deuxième unité de séparation étant en communication fluidique avec la seconde cuve qui contient la fraction déplétée en virus actif.

Une ou plusieurs grandeurs du mélange de la solution basique et de la fraction déplétée en virus actif, telle que le pH, la température, la conductivité, la densité, le pouvoir rotatoire, l'indice de réfraction, l'absorbance ou l'émission d'un rayonnement spectroscopique ou la résonance magnétique nucléaire, de préférence le pH et/ou la température, peuvent être mesurées par un ou plusieurs détecteurs placés dans la deuxième unité de séparation, le détecteur étant de préférence un détecteur en ligne, et/ou placé dans la seconde cuve. Ainsi, si la valeur de la grandeur mesurée (telle que le pH et/ou la température) dévie de la valeur souhaitée de ladite grandeur, cette dernière peut être réajustée. Par exemple, le pH peut être réajusté en ajoutant de la solution basique ou une solution acide dans la deuxième unité de séparation, par la ligne de contournement, de manière à ce qu'elle se mélange avec le mélange de la solution basique et de la fraction déplétée en virus actif.

La fraction déplétée en virus actif est alors traitée par une étape de séparation (par exemple de chromatographie) dans la deuxième unité de séparation. Un rinçage peut être effectué dans la deuxième unité de séparation avant cette étape de séparation (par exemple de chromatographie), en faisant passer un tampon de rinçage dans la deuxième unité de séparation, par la ligne de contournement. Pendant cette étape de rinçage, la deuxième unité de séparation n'est de préférence pas en communication fluidique avec la seconde cuve. Le tampon de rinçage, après passage dans la ligne de contournement, peut être éliminé de l'installation (en étant orienté vers une ligne de collecte des déchets).

Lorsqu'une solution basique est ajoutée à la fraction déplétée en virus actif préalablement ou simultanément à cette étape de séparation (par exemple de chromatographie), et lorsqu'elle est ajoutée en ligne dans la deuxième unité de séparation (la fraction déplétée en virus actif et la solution basique étant passées dans la deuxième unité de séparation simultanément), la fraction déplétée en virus actif à laquelle la solution basique a été ajoutée peut directement passer dans le ou les dispositifs de séparation (par exemple sur la ou les colonnes de chromatographie) de la deuxième unité de séparation pour subir le traitement de séparation, par exemple le traitement chromatographique (dans ce cas, la fraction ne passe pas dans la ligne de contournement). Alternativement, lorsque la solution basique est ajoutée en ligne dans la deuxième unité de séparation (la fraction déplétée en virus actif et la solution basique étant passées dans la deuxième unité de séparation simultanément), la fraction déplétée en virus actif à laquelle la solution basique a été ajoutée en ligne peut passer par la ligne de contournement de la deuxième unité de séparation, une sortie de fluide de la deuxième unité de séparation étant en communication fluidique avec la seconde cuve, et être collectée dans la seconde cuve. Dans ce cas, la fraction déplétée en virus actif et la solution basique sont mélangées dans la ligne de contournement. Un tampon de rinçage peut avantageusement ensuite être passé dans la deuxième unité de séparation, par la ligne de contournement, une sortie de fluide de la deuxième unité de séparation étant en communication fluidique avec la seconde cuve, afin de récupérer dans la seconde cuve la fraction déplétée en virus actif à laquelle la solution basique a été ajoutée restée dans la deuxième unité de séparation. La fraction collectée dans la seconde cuve est ensuite passée dans la deuxième unité de séparation pour subir l'étape de séparation (par exemple de chromatographie).

L'étape de séparation est de préférence une étape de chromatographie mais peut alternativement être une étape de filtration, de centrifugation ou de précipitation.

Avantageusement, le volume de fraction déplétée en virus actif traité par l'étape de séparation dans la deuxième unité de séparation est plus faible que le volume de flux de fluide traité par l'étape de séparation dans la première unité de séparation, pour un volume initial de flux à traiter donné. Dans ces modes de réalisation, la séparation dans la deuxième unité de séparation est donc de préférence plus rapide que la séparation dans la première unité de séparation (pour un volume initial de flux à traiter donné). Ainsi, lorsque les étapes de séparation dans la première unité de séparation et dans la deuxième unité de séparation sont effectuées en continu, c'est-à-dire au moins partiellement en même temps, le procédé comporte nécessairement des temps pendant lesquels la deuxième unité de séparation n'est pas utilisée pour effectuer de séparation (appelés aussi « *temps mort de l'unité de séparation* »). Le procédé selon l'invention présente l'avantage de mettre à profit ce temps mort de la deuxième unité de séparation pour réaliser l'inactivation virale. Cela permet un gain de temps par rapport à un procédé dans lequel l'inactivation virale est effectuée sur la ou les colonnes de la première unité de séparation (allongeant ainsi les temps morts de la première unité de séparation et de la deuxième unité de séparation).

De préférence, la chromatographie est une chromatographie échangeuse d'ions, encore plus préférentiellement une chromatographie échangeuse d'anions ou une chromatographie échangeuse d'anions en mode mixte. Il peut également s'agir d'une chromatographie échangeuse de cations, d'une chromatographie échangeuse de cations en mode mixte ou d'une chromatographie sur charbon actif. La mise en œuvre de telles étapes de chromatographie est bien connue de l'homme du métier.

En sortie de cette étape de séparation (par exemple chromatographie), on collecte une fraction plus enrichie en la substance cible et une autre fraction enrichie en impuretés.

Par « *fraction plus enrichie en substance cible* », on entend une fraction dans laquelle le rapport des concentrations molaires substance cible / impuretés totales est supérieur à celui du flux en entrée de la séparation, par exemple en entrée de chromatographie (correspondant au rapport de concentrations dans la fraction enrichie en substance cible). De manière similaire, l'autre « *fraction enrichie en impuretés* » désigne une fraction dans laquelle le rapport des concentrations molaires substance cible / impuretés totales est inférieur à celui du flux en entrée de la séparation (par exemple en entrée de chromatographie).

De manière particulièrement avantageuse, l'installation selon l'invention comprend une troisième unité de séparation.

La troisième unité de séparation comprend au moins une entrée de fluide et au moins une sortie de fluide, une entrée de fluide de la troisième unité de séparation étant en connexion fluidique avec une sortie de fluide de la deuxième unité de séparation.

La troisième unité de séparation peut être par exemple une unité de chromatographie, incluant des colonnes et/ou des membranes de chromatographie, ou une unité de filtration, de centrifugation, ou de précipitation. La troisième unité de séparation comprend au moins un dispositif de séparation.

De préférence, la troisième unité de séparation est une unité de chromatographie et elle comprend au moins une colonne ou membrane de chromatographie en tant que dispositifs de séparation. Lorsque plusieurs colonnes et/ou membranes sont présentes, elles peuvent être toutes connectées en série, toutes connectées en parallèle, ou l'unité de séparation peut comprendre certaines colonnes et/ou membranes connectées en série et certaines colonnes et/ou membranes connectées en parallèle.

La ou les colonnes et/ou membranes de chromatographie de la troisième unité de séparation contiennent une phase stationnaire. De préférence, toutes les colonnes et/ou membranes de la troisième unité de séparation ont une phase stationnaire identique. Alternativement, les colonnes et/ou membranes peuvent avoir des phases stationnaires différentes.

De manière préférée, la troisième unité de séparation comprend une seule colonne ou membrane de chromatographie.

La phase stationnaire est de préférence une résine échangeuse d'ions. De manière plus préférentielle, il s'agit d'une résine échangeuse de cations. Alternativement, il peut s'agir d'une résine échangeuse de cations en mode mixte, d'une résine échangeuse d'anions ou d'une résine échangeuse d'anions en mode mixte. Dans d'autres modes de réalisation, la phase stationnaire peut être du charbon actif.

De préférence, la ou les entrées de fluide de la troisième unité de séparation sont en connexion fluidique avec un ou plusieurs réservoirs, par exemple un ou plusieurs réservoirs de tampon de rinçage et/ou un réservoir d'éluant ; de préférence, chaque réservoir est en connexion fluidique avec une entrée de fluide différente de la troisième unité de séparation. Les fluides contenus dans ces réservoirs peuvent être apportés à la troisième unité de séparation par une ou plusieurs pompes.

Avantageusement, la fraction plus enrichie en la substance cible collectée en sortie de la deuxième unité de séparation est soumise à un traitement par une étape de chromatographie dans la troisième unité de séparation.

De préférence, la chromatographie est une chromatographie échangeuse d'ions, encore plus préférentiellement une chromatographie échangeuse de cations. Il peut également s'agir d'une chromatographie échangeuse de cations en mode mixte, d'une chromatographie échangeuse d'anions, d'une chromatographie échangeuse d'anions en mode mixte, ou d'une chromatographie sur charbon actif. La mise en œuvre de telles étapes de chromatographie est bien connue de l'homme du métier.

En sortie de la troisième unité de séparation on collecte une fraction purifiée de la substance cible.

Par « *fraction purifiée de substance cible* », on entend une fraction dans laquelle le rapport des concentrations molaires substance cible / impuretés totales est supérieur à celui du flux en entrée de la séparation, par exemple en entrée de chromatographie (correspondant au rapport de concentrations dans la fraction plus enrichie en substance cible).

De manière avantageuse, un rinçage de la deuxième unité de séparation est effectué après l'étape de chromatographie dans la deuxième unité de séparation en faisant passer un tampon de rinçage dans la deuxième unité de séparation, par la ligne de contournement. Pendant cette étape de rinçage, la deuxième unité de séparation n'est de préférence pas en communication fluidique avec la seconde cuve mais est de préférence en communication fluidique avec une ligne de collecte des déchets de sorte à éliminer le tampon de rinçage de l'installation.

Le procédé selon l'invention est de préférence un procédé semi-continu, c'est-à-dire qu'au moins certaines des étapes du procédé sont au moins partiellement effectuées en même temps et au moins certaines des étapes du procédé sont effectuée lorsque l'étape précédente du procédé est terminée. De manière avantageuse, l'étape de séparation (de préférence de chromatographie) dans la première unité de séparation est au moins partiellement effectuée en même temps que l'étape de séparation (de préférence de chromatographie) dans la deuxième unité de séparation.

Les unités de séparation et les cuves peuvent chacune être des équipements réutilisables ou à usage unique.

### Procédé et installation avec une cuve

L'invention concerne également un procédé de purification tel que décrit ci-dessus, dans lequel une seule cuve est utilisée au lieu d'une première cuve et d'une seconde cuve.

Le procédé selon l'invention utilise alors une installation comprenant une cuve, celle-ci étant en connexion fluidique avec une sortie de fluide de la première unité de séparation et avec au moins une entrée de fluide et une sortie de fluide de la deuxième unité de séparation.

Dans ces modes de réalisation, l'invention concerne un procédé de purification d'une substance cible à partir d'un fluide à traiter comprenant au moins une impureté, dans une installation comprenant :
- une première unité de séparation comprenant au moins un dispositif de séparation (par exemple au moins une colonne de chromatographie), une entrée de fluide et une sortie de fluide ;
- une deuxième unité de séparation comprenant au moins un dispositif de séparation (par exemple au moins une colonne de chromatographie), une entrée de fluide, une sortie de fluide et une ligne de contournement contournant l'au moins un dispositif de séparation (par exemple l'au moins une colonne de chromatographie) de la deuxième unité de séparation; et
- une cuve en connexion fluidique avec une sortie de fluide de la première unité de séparation et avec au moins une entrée de fluide et une sortie de fluide de la deuxième unité de séparation ;
le procédé comprenant les étapes suivantes :
- la fourniture d'un flux du fluide à traiter ;
- le traitement du flux de fluide à traiter par une étape de séparation (par exemple de chromatographie) dans la première unité de séparation;
- la collecte d'une fraction enrichie en la substance cible dans la cuve ;
- l'inactivation virale de la fraction enrichie en la substance cible, ladite inactivation virale comprenant :
   ▪ le passage d'une solution d'inactivation virale (par exemple une solution acide) dans la deuxième unité de séparation, par la ligne de contournement ;
   ▪ le mélange de la fraction enrichie en la substance cible et de la solution d'inactivation virale ; et
   ▪ la collecte dans la cuve du mélange de la fraction enrichie en la substance cible et de la solution d'inactivation virale ;
   de manière à obtenir une fraction déplétée en virus actif ;
- le traitement de la fraction déplétée en virus actif par une étape de séparation (par exemple de chromatographie) dans la deuxième unité de séparation ; et
- la collecte en sortie de la deuxième unité de séparation d'une fraction plus enrichie en la substance cible.

L'étape d'inactivation virale peut en outre comprendre une étape de passage de la fraction enrichie en la substance cible collectée dans la cuve dans la deuxième unité de séparation, par la ligne de contournement. De préférence, cette étape est réalisée simultanément à l'étape de passage d'une solution d'inactivation virale (par exemple une solution acide) dans la deuxième unité de séparation, par la ligne de contournement, le mélange de la fraction enrichie en la substance cible et de la solution d'inactivation virale ayant lieu en ligne dans la deuxième unité de séparation, et notamment dans la ligne de contournement. En sortie de la deuxième unité de séparation, le mélange est ensuite collecté dans la cuve.

Dans d'autres modes de réalisation, l'étape d'inactivation virale ne comprend pas d'étape de passage de la fraction enrichie en la substance cible collectée dans la cuve dans la deuxième unité de séparation, par la ligne de contournement. Dans ces modes de réalisation, le mélange de la fraction enrichie en la substance cible et de la solution d'inactivation virale est réalisé dans la cuve.

Mis à part l'utilisation d'une seule cuve au lieu d'une première cuve et d'une seconde cuve, le procédé de purification et l'installation utilisant une cuve peuvent être tels que les procédé/installations décrits ci-dessus avec une première cuve et une seconde cuve. Notamment, les caractéristiques décrites ci-dessus en relation avec la première cuve et la seconde cuve peuvent s'appliquer à cette unique cuve, seules ou en combinaison.

Les modes de réalisation suivants illustrent l'invention sans la limiter.

### Premier procédé de purification d'une substance cible

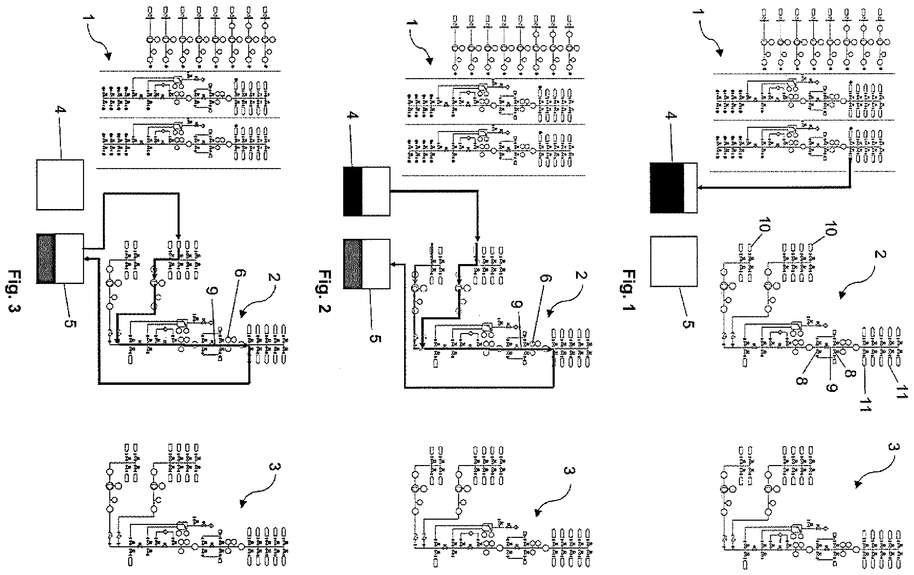

En faisant référence à la **figure 1****,** une installation pour la mise en œuvre du procédé de purification peut comprendre :
- un système chromatographique multicolonne en tant que première unité de séparation 1 comprenant par exemple deux colonnes de chromatographie, de préférence des colonnes d'affinité ;
- une deuxième unité de séparation 2 comprenant une colonne chromatographique, de préférence d'échange d'ions ; des vannes 8 permettent de diriger le fluide au travers de ladite unité de séparation, soit en le faisant passer sur la colonne de chromatographie, soit en court-circuitant la colonne de chromatographie (par la ligne de contournement 9) ;
- une troisième unité de séparation 3 comprenant une colonne chromatographique, de préférence d'échange d'ions ;
- une première cuve 4 ;
- une seconde cuve 5 comprenant des moyens d'agitation.

La deuxième unité de séparation 2 comprend de préférence plusieurs entrées de fluide 10 et plusieurs sorties de fluide 11.

Elle peut comprendre au moins une entrée de fluide pour le fluide venant de la première cuve (pour la fraction enrichie en la substance cible), une entrée de fluide pour une solution acide, une ou plusieurs entrées de fluide pour un tampon de rinçage, une entrée pour une solution basique et/ou une ou plusieurs entrées pour le fluide venant de la seconde cuve (une entrée pour le mélange de la fraction enrichie en la substance cible et de la solution acide, une entrée pour la fraction déplétée en virus et/ou une entrée pour le mélange de la fraction déplétée en virus actif et de la solution basique).

Elle peut comprendre une sortie de fluide pour la fraction enrichie en la substance cible, une sortie pour une solution acide, une sortie pour une solution basique, une ou plusieurs sorties pour un tampon de rinçage (dont une sortie en connexion fluidique avec une ligne de collecte des déchets), une sortie pour le mélange de la fraction enrichie en la substance cible et de la solution acide et/ou une sortie pour le mélange de la fraction déplétée en virus actif et de la solution basique.

Les entrées de fluides ci-dessus peuvent être toutes différentes ou certaines peuvent être les mêmes. Il en est de même pour les sorties de fluide ci-dessus.

De manière préférée, l'installation utilisée pour la mise en œuvre du premier procédé comprend deux entrées de fluide pour un tampon de rinçage, une entrée de fluide pour une solution acide, une entrée de fluide pour une solution basique, une entrée de fluide pour la fraction enrichie en la molécule cible, une entrée de fluide pour la fraction déplétée en virus actif et une entrée de fluide pour le mélange de la fraction enrichie en la substance cible et de la solution acide, ainsi qu'une sortie de fluide pour le tampon de rinçage, connectée à une ligne de collecte des déchets, une sortie de fluide pour le mélange de la fraction enrichie en la substance cible et de la solution acide et pour un tampon de rinçage et une sortie de fluide pour le mélange de la fraction déplétée en virus actif et de la solution basique.

Selon ce procédé, un fluide à traiter comprenant une substance cible est soumis à une étape de chromatographie dans la première unité de séparation 1. A l'issue de cette étape, on collecte dans la première cuve 4 une fraction enrichie en la substance cible, jusqu'à ce que la première cuve 4 soit remplie ou jusqu'à ce que le volume collecté atteigne le volume qui sera traité dans la prochaine étape.

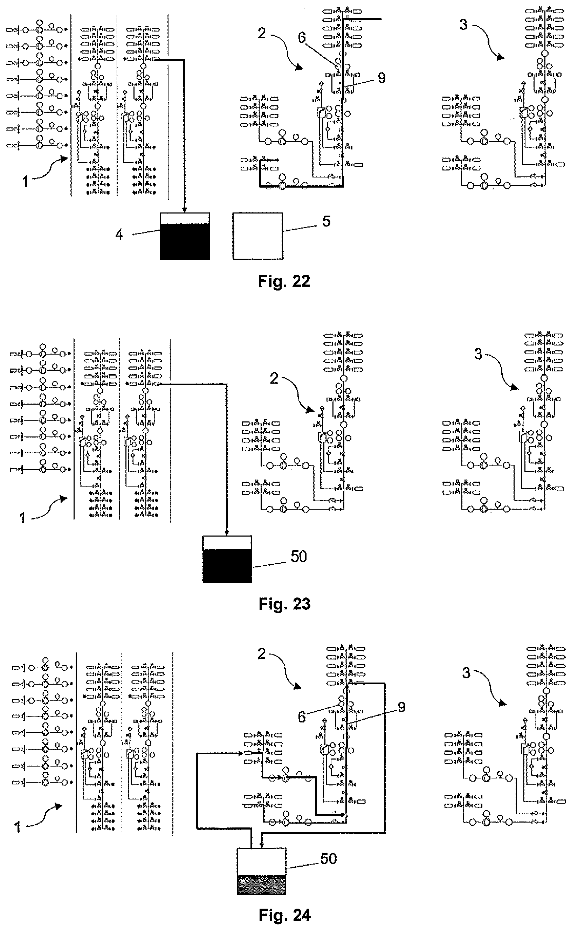

Dans d'autres modes de réalisation, une unique cuve 50 peut être utilisée à la place de la première cuve 4 et de la seconde cuve 5. Dans ces modes de réalisation, l'étape décrite en référence à la **figure 1** est réalisée de la même manière, excepté que l'installation comprend une seule cuve 50 comprenant des moyens d'agitation et que la fraction enrichie en la substance cible est collectée dans la cuve 50, comme montré à la **figure 23****.**

En référence à la **figure 2****,** afin d'ajuster le pH de la fraction enrichie en la substance cible contenue dans la première cuve 4, celle-ci ainsi qu'une solution acide sont toutes deux simultanément injectées dans la deuxième unité de séparation 2 et passent dans la ligne de contournement 9, la colonne de chromatographie étant court-circuitée. La fraction enrichie en la substance cible et la solution acide se mélangent à l'intérieur de la deuxième unité de séparation 2 (mélange en ligne), notamment dans la ligne de contournement 9, et le mélange est collecté dans la seconde cuve 5. Cette étape peut être par exemple effectuée jusqu'à ce que la première cuve 4 soit vide. Le pH du mélange peut être mesuré et contrôlé, par rapport à une valeur de consigne, par un détecteur situé dans la deuxième unité de séparation 2, par exemple le détecteur de pH 6 en ligne, et éventuellement par un détecteur placé dans la seconde cuve 5, et réajusté en modifiant le débit et/ou volume d'entrée de la solution acide.

Dans les modes de réalisation dans lesquels une unique cuve 50 est utilisée à la place de la première cuve 4 et de la seconde cuve 5, l'étape décrite en référence à la **figure 2** est réalisée de la même manière, excepté que les cuves 4 et 5 sont confondues en une cuve 50, comme montré sur la **figure 24** et que, de préférence, la cuve 50 n'est pas vidée. Ainsi, la fraction enrichie en la substance cible contenue dans la cuve 50 à l'issu de l'étape de chromatographie dans la première unité de séparation 1 et la solution acide sont toutes deux simultanément injectées dans la deuxième unité de séparation 2 et passent dans la ligne de contournement 9, la colonne de chromatographie étant court-circuitée. La fraction enrichie en la substance cible et la solution acide se mélangent à l'intérieur de la deuxième unité de séparation 2 (mélange en ligne), notamment dans la ligne de contournement 9, et le mélange est collecté dans la cuve 50.

En référence à la **figure 3****,** le mélange collecté dans la seconde cuve 5 est repassé dans la deuxième unité de séparation 2 et passe dans la ligne de contournement 9, la colonne de chromatographie étant toujours court-circuitée, puis est collecté de nouveau dans la seconde cuve 5. Cette étape permet de récupérer dans la seconde cuve 5 la portion de fraction enrichie en la substance cible qui pourrait rester à l'intérieur de la deuxième unité de séparation 2 et permet également d'homogénéiser le mélange. Le pH du mélange peut être mesuré et contrôlé, par rapport à une valeur de consigne, par un détecteur situé dans la deuxième unité de séparation 2, par exemple le détecteur de pH 6 en ligne, et éventuellement par un détecteur placé dans la seconde cuve 5, et peut être réajusté en modifiant le débit et/ou volume d'entrée de la solution acide.

Dans les modes de réalisation dans lesquels une unique cuve 50 est utilisée à la place de la première cuve 4 et de la seconde cuve 5, l'étape décrite en référence à la **figure 3** est réalisée de la même manière, excepté que la seconde cuve 5 est remplacée par la cuve 50.

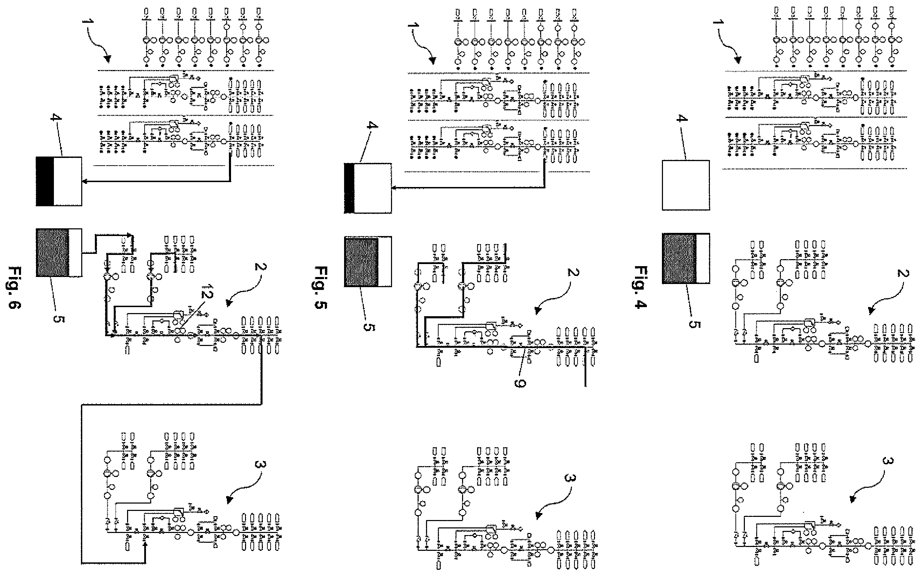

En référence à la **figure 4****,** le mélange est laissé à incuber, sous agitation, pendant une certaine durée, par exemple de 30 minutes environ, dans la seconde cuve 5 afin d'effectuer l'inactivation virale. On obtient une fraction déplétée en virus actif. Le pH du mélange dans la seconde cuve 5 peut être mesuré en continu par un détecteur placé dans la seconde cuve 5.

Dans les modes de réalisation dans lesquels une unique cuve 50 est utilisée à la place de la première cuve 4 et de la seconde cuve 5, l'étape décrite en référence à la **figure 4** est réalisée de la même manière, excepté que la seconde cuve 5 est remplacée par la cuve 50.

En référence à la **figure 5****,** un nouveau flux de fluide à traiter subit une étape de chromatographie dans la première unité de séparation 1 et une nouvelle fraction enrichie en la substance cible est collectée dans la première cuve 4 en sortie de cette étape de chromatographie, jusqu'à ce que la première cuve 4 soit remplie ou jusqu'à ce que le volume collecté atteigne le volume qui sera traité dans la prochaine étape. Dans le même temps, un rinçage de la deuxième unité de séparation 2 est effectué en passant du tampon de rinçage dans la deuxième unité de séparation 2 et notamment dans la ligne de contournement 9, la colonne de chromatographie étant court-circuitée. Le tampon de rinçage peut être par exemple éliminé en tant que déchet en sortie. Alternativement ou en addition, au moins une partie du tampon de rinçage peut être récupéré dans la seconde cuve 5, la deuxième unité de séparation 2 étant alors en communication fluidique avec la seconde cuve 5. Cela permet de récupérer dans la seconde cuve 5 le mélange qui pourrait être resté à l'intérieur de la deuxième unité de séparation 2. L'orientation du tampon de rinçage, après son passage dans la ligne de contournement 9, vers une ligne de collecte des déchets ou vers la seconde cuve 5 peut être décidée sur l'atteinte d'une valeur de consigne de pH, ou de conductivité, ou d'un volume passé de tampon de rinçage ou encore sur une temporisation.

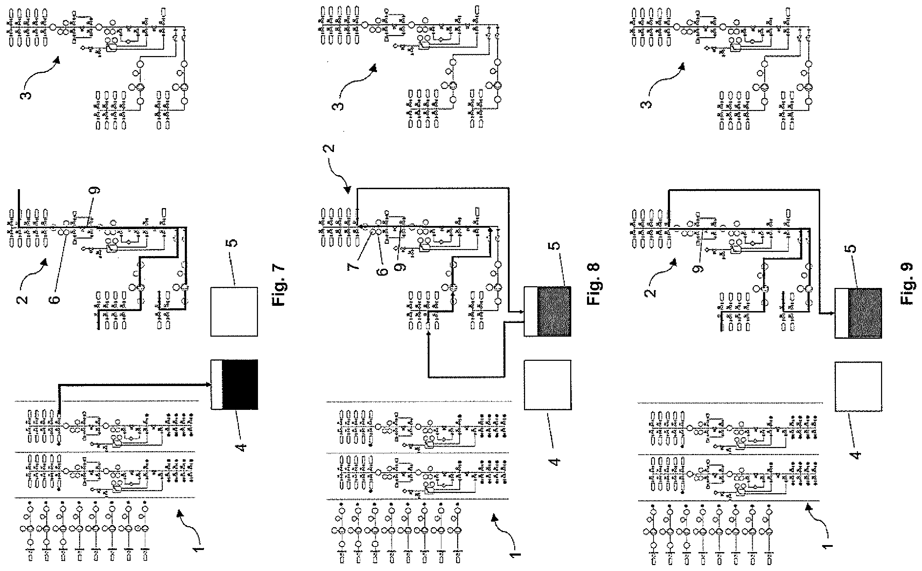

Dans les modes de réalisation dans lesquels une unique cuve 50 est utilisée à la place de la première cuve 4 et de la seconde cuve 5, l'étape décrite en référence à la **figure 5** est réalisée de la même manière, excepté que le traitement par une étape de chromatographie dans la première unité de séparation 1 d'un nouveau flux n'est pas réalisé en même temps que l'étape de rinçage mais est effectué après le traitement chromatographique par la deuxième unité de séparation 2 décrit en référence à la **figure 6****,** en même temps que l'étape de rinçage décrite en référence à la **figure 7**. En référence à la **figure 6****,** une fois l'inactivation virale achevée, afin d'ajuster le pH de la fraction déplétée en virus actif pour le prochain traitement de ladite fraction, celle-ci et une solution basique sont toutes deux simultanément injectées dans la deuxième unité de séparation 2, la colonne de chromatographie étant en ligne (c'est-à-dire non court-circuitée). La fraction déplétée en virus actif et la solution basique se mélangent à l'intérieur de la deuxième unité de séparation 2 (mélange en ligne). Le pH du mélange peut être mesuré et contrôlé, par rapport à une valeur de consigne, par un détecteur situé dans la deuxième unité de séparation 2, par exemple le détecteur de pH 12 en ligne, et peut être réajusté en modifiant le débit et/ou volume d'entrée de la solution basique. Le mélange est passé sur la colonne de chromatographie de la deuxième unité de séparation 2 pour subir une étape de chromatographie. A l'issue de cette étape de chromatographie, on récupère une fraction plus enrichie en la substance cible. Cette fraction est ensuite amenée à la troisième unité de séparation 3, dans laquelle elle subit une étape de chromatographie, et en sortie de laquelle on récupère une fraction purifiée en la substance cible. Ces étapes sont effectuées par exemple jusqu'à ce que la seconde cuve 5 soit vide.

Dans les modes de réalisation dans lesquels une unique cuve 50 est utilisée à la place de la première cuve 4 et de la seconde cuve 5, l'étape décrite en référence à la **figure 6** est réalisée de la même manière, excepté que la seconde cuve 5 est remplacée par la cuve 50.

En référence à la **figure 7****,** un rinçage est effectué en passant du tampon de rinçage dans la deuxième unité de séparation 2 et notamment dans la ligne de contournement 9, la colonne de chromatographie étant court-circuitée. Le pH du tampon de rinçage peut être mesuré et contrôlé, par rapport à une valeur de consigne, par un détecteur situé dans la deuxième unité de séparation 2, par exemple le détecteur de pH 6 en ligne. Le débit et/ou volume d'entrée du tampon de rinçage peut alors être modifié en fonction du pH mesuré.

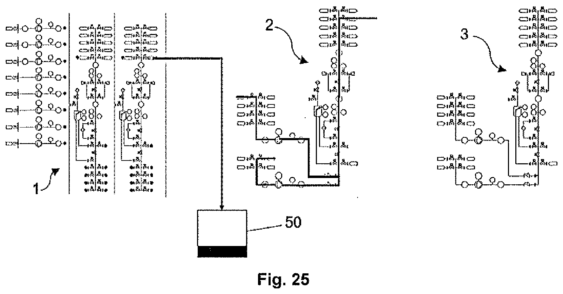

Dans les modes de réalisation dans lesquels une unique cuve 50 est utilisée à la place de la première cuve 4 et de la seconde cuve 5, l'étape décrite en référence à la **figure 7** est réalisée de la même manière, excepté que, dans le même temps, un nouveau flux de fluide à traiter subit une étape de chromatographie dans la première unité de séparation 1 et une nouvelle fraction enrichie en la substance cible est collectée dans la cuve 50 en sortie de cette étape de chromatographie, jusqu'à ce que la cuve 50 soit remplie ou jusqu'à ce que le volume collecté atteigne le volume qui sera traité dans la prochaine étape, comme montré à la **figure 25****.**

Le cycle est ensuite répété à partir de l'étape d'ajustement du pH, avec la solution acide, de la fraction enrichie en la substance cible contenue dans la première cuve 4 telle que montrée dans la **figure 2****,** ou, dans les modes de réalisation dans lesquels une unique cuve 50 est utilisée à la place de la première cuve 4 et de la seconde cuve 5, le cycle est répété à partir de l'étape d'ajustement du pH, avec la solution acide, de la fraction enrichie en la substance cible contenue dans la cuve 50 telle que montrée dans la **figure 24****.**

### Deuxième procédé de purification d'une substance cible

Le deuxième procédé de purification peut utiliser la même installation décrite ci-dessus en référence à la **figure 1** et au premier procédé.

De manière préférée, l'installation utilisée pour la mise en œuvre du deuxième procédé comprend deux entrées de fluide pour un tampon de rinçage, une entrée de fluide pour une solution acide, une entrée de fluide pour une solution basique, une entrée de fluide pour la fraction enrichie en la molécule cible, une entrée de fluide pour la fraction déplétée en virus actif et pour le mélange de la fraction déplétée en virus actif et de la solution basique et une entrée de fluide pour le mélange de la fraction enrichie en la substance cible et de la solution acide, ainsi qu'une sortie de fluide pour le tampon de rinçage, connectée à une ligne de collecte des déchets, une sortie de fluide pour le mélange de la fraction enrichie en la substance cible et de la solution acide et pour un tampon de rinçage et une sortie de fluide pour le mélange de la fraction déplétée en virus actif et de la solution basique.

Selon ce procédé, un fluide à traiter comprenant une substance cible est soumis à une étape de chromatographie dans la première unité de séparation 1. A l'issue de cette étape, on collecte dans la première cuve 4 une fraction enrichie en la substance cible, jusqu'à ce que la première cuve 4 soit remplie ou jusqu'à ce que le volume collecté atteigne le volume qui sera traité dans la prochaine étape.

Dans d'autres modes de réalisation, une unique cuve 50 peut être utilisée à la place de la première cuve 4 et de la seconde cuve 5. Dans ces modes de réalisation, l'étape décrite en référence à la **figure 1** est réalisée de la même manière, excepté que l'installation comprend une seule cuve 50 comprenant des moyens d'agitation et que la fraction enrichie en la substance cible est collectée dans la cuve 50, comme montré à la **figure 23**. En référence à la **figure 2****,** afin d'ajuster le pH de la fraction enrichie en la substance cible contenue dans la première cuve 4, celle-ci ainsi qu'une solution acide sont toutes deux simultanément injectées dans la deuxième unité de séparation 2 et passent dans la ligne de contournement 9, la colonne de chromatographie étant court-circuitée. La fraction enrichie en la substance cible et la solution acide se mélange à l'intérieur de la deuxième unité de séparation 2 (mélange en ligne) et le mélange est collecté dans la seconde cuve 5. Cette étape est effectuée par exemple jusqu'à ce que la première cuve 4 soit vide. Le pH du mélange peut être mesuré et contrôlé, par rapport à une valeur de consigne, par un détecteur situé dans la deuxième unité de séparation 2, par exemple le détecteur de pH 6 en ligne, et éventuellement par un détecteur placé dans la seconde cuve 5, et réajusté en modifiant le débit et/ou volume d'entrée de la solution acide.

Dans les modes de réalisation dans lesquels une unique cuve 50 est utilisée à la place de la première cuve 4 et de la seconde cuve 5, l'étape décrite en référence à la **figure 2** est réalisée de la même manière, excepté que les cuves 4 et 5 sont confondues en une cuve 50, comme montré sur la **figure 24** et que, de préférence, la cuve 50 n'est pas vidée. Ainsi, la fraction enrichie en la substance cible contenue dans la cuve 50 à l'issu de l'étape de chromatographie dans la première unité de séparation 1 et la solution acide sont toutes deux simultanément injectées dans la deuxième unité de séparation 2 et passent dans la ligne de contournement 9, la colonne de chromatographie étant court-circuitée. La fraction enrichie en la substance cible et la solution acide se mélangent à l'intérieur de la deuxième unité de séparation 2 (mélange en ligne), notamment dans la ligne de contournement 9, et le mélange est collecté dans la cuve 50. En référence à la **figure 8****,** le mélange collecté dans la seconde cuve 5 est repassé dans la deuxième unité de séparation 2 et passe dans la ligne de contournement 9, la colonne de chromatographie étant toujours court-circuitée, puis est collecté de nouveau dans la seconde cuve 5. Ces étapes (de recirculation dans la deuxième unité de séparation 2 et de collecte dans la seconde cuve 5) sont répétées ou mises en œuvre en continu pendant une certaine durée, par exemple 30 minutes environ, afin d'effectuer l'inactivation virale. On obtient une fraction déplétée en virus actif. Un contrôle du pH et de la température du mélange peut être effectué à chaque passage du mélange dans la seconde unité de séparation 2 ou en continu, selon le cas, à l'aide de détecteurs en ligne de pH 6 et de température 7. Ainsi, en cas de mesure d'une déviation par rapport à une valeur de pH et/ou de température de consigne, le pH et/ou la température peuvent être réajustés.

Dans les modes de réalisation dans lesquels une unique cuve 50 est utilisée à la place de la première cuve 4 et de la seconde cuve 5, l'étape décrite en référence à la **figure 8** est réalisée de la même manière, excepté que la seconde cuve 5 est remplacée par la cuve 50.

En référence à la **figure 9****,** un tampon de rinçage est ensuite passé dans la deuxième unité de séparation 2, et plus particulièrement dans la ligne de contournement 9, la colonne de chromatographie étant court-circuitée et la deuxième unité de séparation 2 étant en communication fluidique avec la seconde cuve 5. Cela permet de récupérer dans la seconde cuve 5 le mélange qui pourrait être resté à l'intérieur de la deuxième unité de séparation 2. Idéalement, le volume du tampon de rinçage passé dans la deuxième unité de séparation 2 pendant que celle-ci est en communication fluidique avec la seconde cuve 5 est supérieur au volume interne des tuyaux de la deuxième unité de séparation 2.

Dans les modes de réalisation dans lesquels une unique cuve 50 est utilisée à la place de la première cuve 4 et de la seconde cuve 5, l'étape décrite en référence à la **figure 9** est réalisée de la même manière, excepté que la seconde cuve 5 est remplacée par la cuve 50.

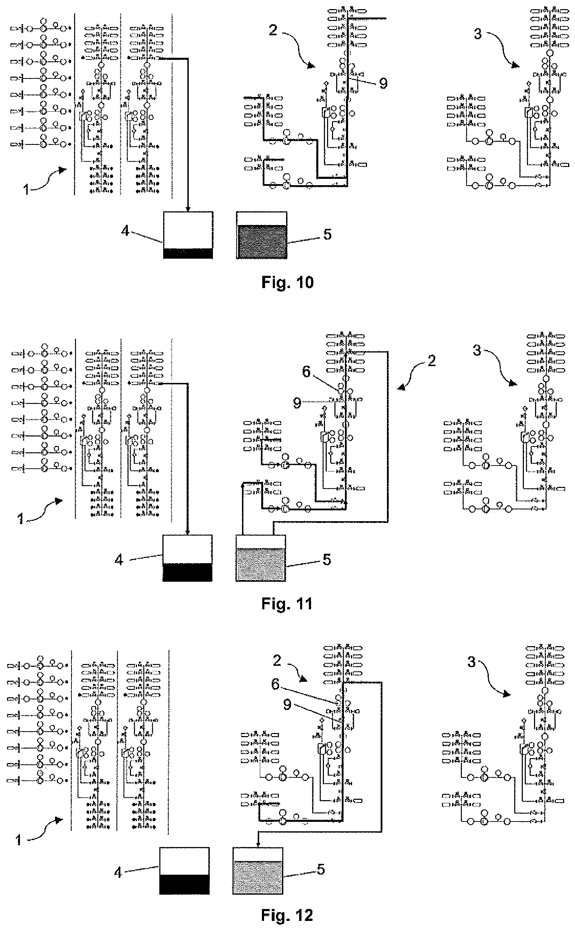

En référence à la **figure 10****,** un nouveau flux de fluide à traiter subit une étape de chromatographie dans la première unité de séparation 1 et une nouvelle fraction enrichie en la substance cible est collectée dans la première cuve 4, en sortie de cette étape de chromatographie, jusqu'à ce que la première cuve 4 soit remplie ou jusqu'à ce que le volume collecté atteigne le volume qui sera traité dans la prochaine étape. Dans le même temps, un rinçage de la deuxième unité de séparation 2 est effectué en passant du tampon de rinçage dans la deuxième unité de séparation 2 et notamment dans la ligne de contournement 9, la colonne de chromatographie étant court-circuitée, et la deuxième unité de séparation n'étant pas en communication fluidique avec la seconde cuve 5 mais avec une ligne de collecte de déchets. L'orientation du tampon de rinçage, après son passage dans la ligne de contournement 9, vers une ligne de collecte des déchets ou vers la seconde cuve 5 peut être décidée sur l'atteinte d'une valeur de consigne de pH, ou de conductivité, ou d'un volume passé de tampon de rinçage ou encore sur une temporisation.

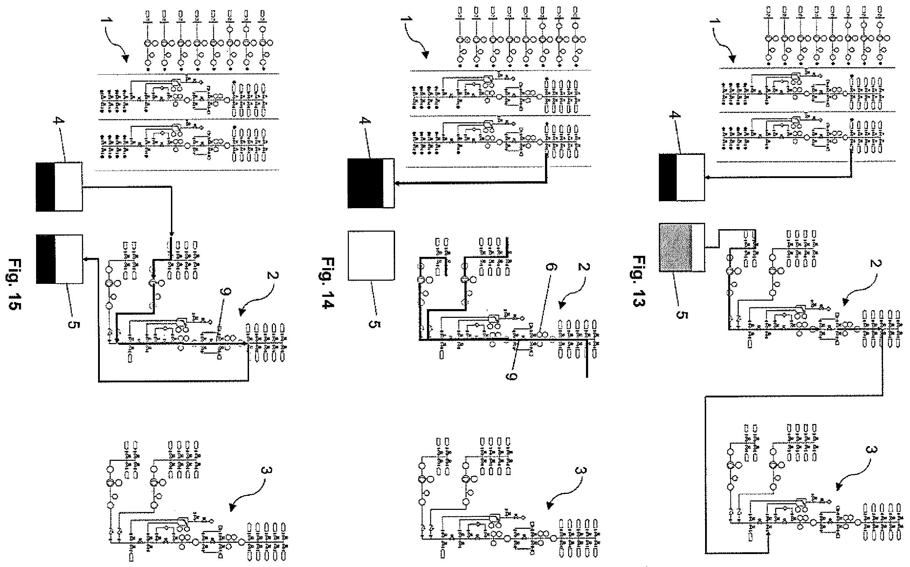

Dans les modes de réalisation dans lesquels une unique cuve 50 est utilisée à la place de la première cuve 4 et de la seconde cuve 5, l'étape décrite en référence à la **figure 10** est réalisée de la même manière, excepté que le traitement par une étape de chromatographie dans la première unité de séparation 1 d'un nouveau flux n'est pas réalisé en même temps que l'étape de rinçage mais est effectué après le traitement chromatographique par la deuxième unité de séparation 2 décrit en référence à la **figure 13****,** en même temps que l'étape de rinçage décrite en référence à la **figure 14**. En référence à la **figure 11****,** afin d'ajuster le pH de la fraction déplétée en virus actif pour le prochain traitement de ladite fraction, celle-ci et une solution basique sont toutes deux simultanément injectées dans la deuxième unité de séparation 2 et passent dans la ligne de contournement 9, la colonne de chromatographie étant court-circuitée et la deuxième unité de séparation 2 étant en communication fluidique avec la seconde cuve 5. La fraction déplétée en virus actif et la solution basique se mélangent à l'intérieur de la deuxième unité de séparation 2 (mélange en ligne) et le mélange est collecté dans la seconde cuve 5. Le pH du mélange peut être mesuré et contrôlé, par rapport à une valeur de consigne, par un détecteur situé dans la deuxième unité de séparation 2, par exemple le détecteur de pH 6 en ligne, et éventuellement par un détecteur placé dans la seconde cuve 5, et réajusté en modifiant le débit et/ou volume d'entrée de la solution basique. Le mélange collecté dans la seconde cuve 5 peut être optionnellement repassé dans la deuxième unité de séparation 2, la colonne de chromatographie étant court-circuitée, et collecté de nouveau dans la seconde cuve, une ou plusieurs fois ou en continu. Cela permet de contrôler ou recontrôler le pH du mélange, par exemple à l'aide d'un détecteur de pH 6 en ligne, et de l'ajuster de manière précise avant le prochain traitement chromatographique.

Dans les modes de réalisation dans lesquels une unique cuve 50 est utilisée à la place de la première cuve 4 et de la seconde cuve 5, l'étape décrite en référence à la **figure 11** est réalisée de la même manière, excepté que la seconde cuve 5 est remplacée par la cuve 50.

En référence à la **figure 12****,** un tampon de rinçage est ensuite passé dans la deuxième unité de séparation 2, dans la ligne de contournement 9, la colonne de chromatographie étant court-circuitée et la deuxième unité de séparation 2 étant en communication fluidique avec la seconde cuve 5. Cela permet de récupérer dans la seconde cuve 5 le mélange qui pourrait être resté à l'intérieur de la deuxième unité de séparation 2. Le pH du tampon de rinçage peut être mesuré et contrôlé, par rapport à une valeur de consigne, par un détecteur situé dans la deuxième unité de séparation 2, par exemple le détecteur de pH 6 en ligne. Le débit et/ou volume d'entrée du tampon de rinçage peut alors être modifié en fonction du pH mesuré.

Dans les modes de réalisation dans lesquels une unique cuve 50 est utilisée à la place de la première cuve 4 et de la seconde cuve 5, l'étape décrite en référence à la **figure 12** est réalisée de la même manière, excepté que la seconde cuve 5 est remplacée par la cuve 50.

En référence à la **figure 13****,** le mélange contenu dans la seconde cuve 5 est passé dans la deuxième unité de séparation 2, y compris dans la colonne de chromatographie qui est en ligne, pour subir une étape de chromatographie. A l'issu de cette étape de chromatographie, on récupère une fraction plus enrichie en la substance cible. Cette fraction est ensuite amenée à la troisième unité de séparation 3, dans laquelle elle subit une étape de chromatographie, et en sortie de laquelle on récupère une fraction purifiée en la substance cible. Ces étapes sont effectuées par exemple jusqu'à ce que la seconde cuve 5 soit vide.

Dans les modes de réalisation dans lesquels une unique cuve 50 est utilisée à la place de la première cuve 4 et de la seconde cuve 5, l'étape décrite en référence à la **figure 13** est réalisée de la même manière, excepté que la seconde cuve 5 est remplacée par la cuve 50.

En référence à la **figure 14****,** un rinçage est effectué en passant du tampon de rinçage dans la deuxième unité de séparation 2 et notamment dans la ligne de contournement 9, la colonne de chromatographie étant court-circuitée. Le pH du tampon de rinçage peut être mesuré et contrôlé, par rapport à une valeur de consigne, par un détecteur situé dans la deuxième unité de séparation 2, par exemple le détecteur de pH 6 en ligne. Le débit et/ou volume d'entrée du tampon de rinçage peut alors être modifié en fonction du pH mesuré.

Dans les modes de réalisation dans lesquels une unique cuve 50 est utilisée à la place de la première cuve 4 et de la seconde cuve 5, l'étape décrite en référence à la **figure 14** est réalisée de la même manière, excepté que, dans le même temps, un nouveau flux de fluide à traiter subit une étape de chromatographie dans la première unité de séparation 1 et une nouvelle fraction enrichie en la substance cible est collectée dans la cuve 50 en sortie de cette étape de chromatographie, jusqu'à ce que la cuve 50 soit remplie ou jusqu'à ce que le volume collecté atteigne le volume qui sera traité dans la prochaine étape, comme montré à la **figure 25****.**

Le cycle est ensuite répété à partir de l'étape d'ajustement du pH, avec la solution acide, de la fraction enrichie en la substance cible contenue dans la première cuve 4 telle que montrée dans la **figure 2****,** ou, dans les modes de réalisation dans lesquels une unique cuve 50 est utilisée à la place de la première cuve 4 et de la seconde cuve 5, le cycle est répété à partir de l'étape d'ajustement du pH, avec la solution acide, de la fraction enrichie en la substance cible contenue dans la cuve 50 telle que montrée dans la **figure 24****.**

### Troisième procédé de purification d'une substance cible

Le troisième procédé de purification peut utiliser la même installation décrite ci-dessus en référence à la **figure 1** et au premier procédé.

De manière préférée, l'installation utilisée pour la mise en œuvre du deuxième procédé comprend deux entrées de fluide pour un tampon de rinçage, une entrée de fluide pour une solution acide, une entrée de fluide pour une solution basique, une entrée de fluide pour la fraction enrichie en la molécule cible et une entrée de fluide pour le mélange de la fraction déplétée en virus actif et de la solution basique, ainsi qu'une sortie de fluide pour le tampon de rinçage, connectée à une ligne de collecte des déchets, une sortie de fluide pour la fraction enrichie en la substance cible, pour une solution acide et pour une solution basique et une sortie de fluide pour le mélange de la fraction déplétée en virus actif et de la solution basique.

Selon ce procédé, un fluide à traiter comprenant une substance cible est soumis à une étape de chromatographie dans la première unité de séparation 1. A l'issu de cette étape, on collecte dans la première cuve 4 une fraction enrichie en la substance cible, jusqu'à ce que la première cuve 4 soit remplie ou jusqu'à ce que le volume collecté atteigne le volume qui sera traité dans la prochaine étape.

Dans d'autres modes de réalisation, une unique cuve 50 peut être utilisée à la place de la première cuve 4 et de la seconde cuve 5. Dans ces modes de réalisation, l'étape décrite en référence à la **figure 1** est réalisée de la même manière, excepté que l'installation comprend une seule cuve 50 comprenant des moyens d'agitation, que la fraction enrichie en la substance cible est collectée dans la cuve 50, comme montré à la **figure 23** et que, de préférence l'installation ne comprend ni d'entrée de fluide ni de sortie de fluide pour la fraction enrichie en la molécule cible.

En référence à la **figure 15****,** la fraction enrichie en la substance cible contenue dans la première cuve 4 est passée dans la deuxième unité de séparation 2, en passant dans la ligne de contournement 9, la colonne de chromatographie étant court-circuitée et la deuxième unité de séparation 2 étant en communication fluidique avec la seconde cuve 5, et est collectée dans la seconde cuve 5.

Dans les modes de réalisation dans lesquels une unique cuve 50 est utilisée à la place de la première cuve 4 et de la seconde cuve 5, l'étape décrite en référence à la **figure 15** n'est pas effectuée.

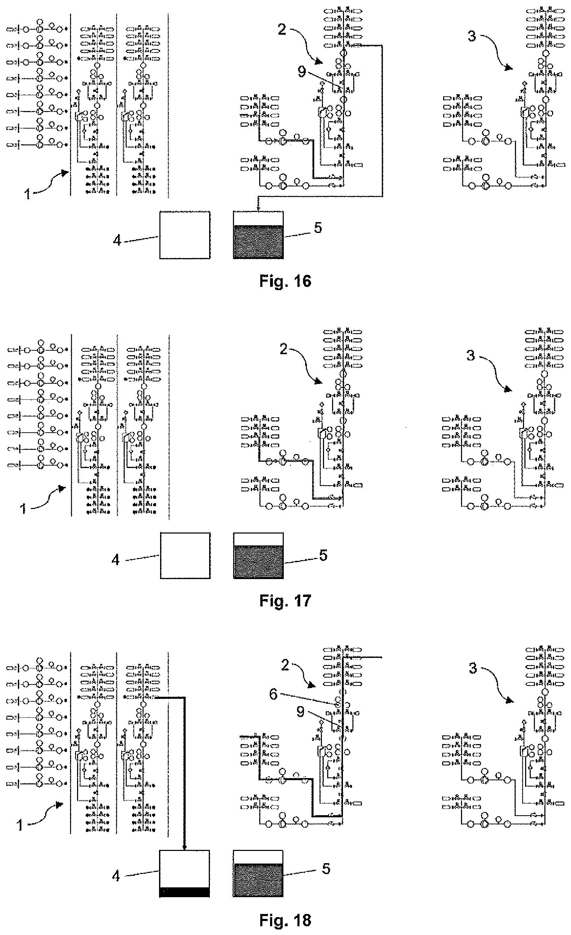

En référence à la **figure 16****,** afin d'ajuster le pH de la fraction enrichie en la substance cible contenue dans la seconde cuve 5, une solution acide est passée dans la deuxième unité de séparation 2, en passant dans la ligne de contournement 9, la colonne de chromatographie étant court-circuitée et la deuxième unité de séparation 2 étant en communication fluidique avec la seconde cuve 5. La solution acide est mélangée dans la seconde cuve 5 avec la fraction enrichie en la substance cible contenue dans la seconde cuve 5, à l'aide des moyes d'agitation de la seconde cuve 5. Le pH du mélange peut être mesuré et contrôlé, par rapport à une valeur de consigne, par un détecteur de pH situé dans la seconde cuve 5, et le pH peut être réajusté en modifiant le débit et/ou volume d'entrée de la solution acide.

Dans les modes de réalisation dans lesquels une unique cuve 50 est utilisée à la place de la première cuve 4 et de la seconde cuve 5, l'étape décrite en référence à la **figure 16** est réalisée de la même manière, excepté que la seconde cuve 5 est remplacée par la cuve 50.

En référence à la **figure 17****,** le mélange est laissé à incuber, sous agitation, pendant une certaine durée, par exemple de 30 minutes environ, dans la seconde cuve 5 afin d'effectuer l'inactivation virale. On obtient une fraction déplétée en virus actif.

Dans les modes de réalisation dans lesquels une unique cuve 50 est utilisée à la place de la première cuve 4 et de la seconde cuve 5, l'étape décrite en référence à la **figure 17** est réalisée de la même manière, excepté que la seconde cuve 5 est remplacée par la cuve 50.

En référence à la **figure 18****,** un nouveau flux de fluide à traiter subit une étape de chromatographie dans la première unité de séparation 1 et une nouvelle fraction enrichie en la substance cible est collectée dans la première cuve 4, en sortie de cette étape de chromatographie, jusqu'à ce que la première cuve 4 soit remplie ou jusqu'à ce que le volume collecté atteigne le volume qui sera traité dans la prochaine étape. Dans le même temps, un rinçage de la deuxième unité de séparation 2 est effectué en passant du tampon de rinçage dans la deuxième unité de séparation 2, en passant dans la ligne de contournement 9, la colonne de chromatographie étant court-circuitée. Le tampon de rinçage entraînant la solution acide restant dans le circuit peut ensuite être éliminé en sortie comme déchet. Le pH du tampon de rinçage peut être mesuré et contrôlé, par rapport à une valeur de consigne, par un détecteur situé dans la deuxième unité de séparation 2, par exemple le détecteur de pH 6 en ligne. Le débit et/ou volume d'entrée du tampon de rinçage peut alors être modifié en fonction du pH mesuré.

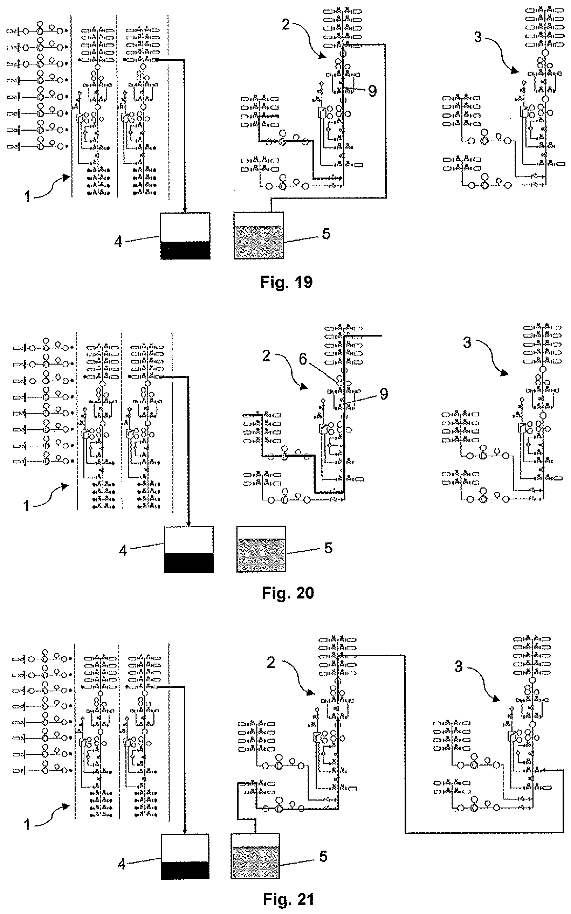

Dans les modes de réalisation dans lesquels une unique cuve 50 est utilisée à la place de la première cuve 4 et de la seconde cuve 5, l'étape décrite en référence à la **figure 18** est réalisée de la même manière, excepté que le traitement par une étape de chromatographie dans la première unité de séparation 1 d'un nouveau flux n'est pas réalisé en même temps que l'étape de rinçage mais est effectué après le traitement chromatographique par la deuxième unité de séparation 2 décrit en référence à la **figure 21****,** en même temps que l'étape de rinçage décrite en référence à la **figure 22****.**

En référence à la **figure 19****,** afin d'ajuster le pH de la fraction déplétée en virus actif pour le prochain traitement de ladite fraction, une solution basique est passée dans la deuxième unité de séparation 2, en passant par la ligne de contournement 9, la colonne de chromatographie étant court-circuitée et la deuxième unité de séparation 2 étant en communication fluidique avec la seconde cuve 5. La solution basique est mélangée dans la seconde cuve 5 avec la fraction déplétée en virus actif contenue dans la seconde cuve 5, à l'aide des moyens d'agitation de la seconde cuve 5. Le pH du mélange peut être mesuré et contrôlé, par rapport à une valeur de consigne, par un détecteur de pH situé dans la seconde cuve 5, et le pH peut être réajusté en modifiant le débit et/ou volume d'entrée de la solution basique.

Dans les modes de réalisation dans lesquels une unique cuve 50 est utilisée à la place de la première cuve 4 et de la seconde cuve 5, l'étape décrite en référence à la **figure 19** est réalisée de la même manière, excepté que la seconde cuve 5 est remplacée par la cuve 50.

En référence à la **figure 20****,** un rinçage est effectué en passant du tampon de rinçage dans la deuxième unité de séparation 2, en passant par la ligne de contournement 9, la colonne de chromatographie étant court-circuitée. Le tampon de rinçage entraînant la solution basique restant dans le circuit peut ensuite être éliminé en sortie comme déchet. Le pH du tampon de rinçage peut être mesuré et contrôlé, par rapport à une valeur de consigne, par un détecteur situé dans la deuxième unité de séparation 2, par exemple le détecteur de pH 6 en ligne. Le débit et/ou volume d'entrée du tampon de rinçage peut alors être modifié en fonction du pH mesuré.

Dans les modes de réalisation dans lesquels une unique cuve 50 est utilisée à la place de la première cuve 4 et de la seconde cuve 5, l'étape décrite en référence à la **figure 20** est réalisée de la même manière, excepté que la seconde cuve 5 est remplacée par la cuve 50.

En référence à la **figure 21****,** le mélange contenu dans la seconde cuve 5, est passé dans la deuxième unité de séparation 2, y compris dans la colonne de chromatographie qui est en ligne, pour subir une étape de chromatographie dans celle-ci. A l'issue de cette étape de chromatographie, on récupère une fraction plus enrichie en la substance cible. Cette fraction est ensuite amenée à la troisième unité de séparation 3, dans laquelle elle subit une étape de chromatographie, et en sortie de laquelle on récupère une fraction purifiée en la substance cible. Ces étapes sont effectuées par exemple jusqu'à ce que la seconde cuve 5 soit vide.

Dans les modes de réalisation dans lesquels une unique cuve 50 est utilisée à la place de la première cuve 4 et de la seconde cuve 5, l'étape décrite en référence à la **figure 21** est réalisée de la même manière, excepté que la seconde cuve 5 est remplacée par la cuve 50.

En référence à la **figure 22****,** un rinçage est effectué en passant du tampon de rinçage dans la deuxième unité de séparation 2, en passant par la ligne de contournement 9, la colonne de chromatographie étant court-circuitée. Le tampon de rinçage entraînant le mélange restant dans le circuit peut ensuite être éliminé en sortie comme déchet. Le pH du tampon de rinçage peut être mesuré et contrôlé, par rapport à une valeur de consigne, par un détecteur situé dans la deuxième unité de séparation 2, par exemple le détecteur de pH 6 en ligne. Le débit et/ou volume d'entrée du tampon de rinçage peut alors être modifié en fonction du pH mesuré.

Dans les modes de réalisation dans lesquels une unique cuve 50 est utilisée à la place de la première cuve 4 et de la seconde cuve 5, l'étape décrite en référence à la **figure 22** est réalisée de la même manière, excepté que, dans le même temps, un nouveau flux de fluide à traiter subit une étape de chromatographie dans la première unité de séparation 1 et une nouvelle fraction enrichie en la substance cible est collectée dans la cuve 50 en sortie de cette étape de chromatographie, jusqu'à ce que la cuve 50 soit remplie ou jusqu'à ce que le volume collecté atteigne le volume qui sera traité dans la prochaine étape.

Le cycle est ensuite répété à partir de l'étape de passage de la fraction enrichie en la substance cible contenue dans la première cuve 4 dans la seconde cuve 5 à travers la deuxième unité de séparation 2, telle que montrée dans la **figure 15****,** ou, dans les modes de réalisation dans lesquels une unique cuve 50 est utilisée à la place de la première cuve 4 et de la seconde cuve 5, le cycle est répété à partir de l'étape de passage de la solution acide dans la deuxième unité de séparation 2 par la ligne de contournement 9.

### Programme d'ordinateur

Un autre objet de l'invention consiste en un programme d'ordinateur comprenant des instructions de code de programme pour l'exécution des étapes du procédé selon l'invention lorsque ledit programme est exécuté sur un ordinateur.

L'invention concerne aussi un support de stockage lisible par ordinateur sur lequel est enregistré un programme d'ordinateur tel que défini ci-dessus.

L'invention concerne aussi un système comprenant un processeur couplé à une mémoire sur laquelle est enregistré un programme d'ordinateur tel que défini ci-dessus. Ledit système peut également comprendre l'installation telle que décrite ci-dessus ou être uniquement un système de commande, connecté à l'installation et distinct de celle-ci.

## Revendications

1. Procédé de purification d'une substance cible à partir d'un fluide à traiter comprenant au moins une impureté, dans une installation comprenant :
- une première unité de séparation (1) comprenant au moins une colonne de chromatographie, une entrée de fluide et une sortie de fluide ;
- une deuxième unité de séparation (2) comprenant au moins une colonne de chromatographie, une entrée de fluide (10), une sortie de fluide (11) et une ligne de contournement (9) reliée à au moins une entrée de fluide (10) de la deuxième unité de séparation à l'une de ses extrémités et à au moins une sortie de fluide (11) de la deuxième unité de séparation à l'autre de ses extrémités et contournant la ou les colonnes de chromatographie de la deuxième unité de séparation (2) ;
- une première cuve (4) en connexion fluidique avec une sortie de fluide de la première unité de séparation (1) et avec une entrée de fluide (10) de la deuxième unité de séparation (2) ; et
- une seconde cuve (5) en connexion fluidique avec au moins une entrée de fluide (10) et une sortie de fluide (11) de la deuxième unité de séparation (2) ;
le procédé comprenant les étapes suivantes :
- la fourniture d'un flux du fluide à traiter ;
- le traitement du flux de fluide à traiter par une étape de chromatographie dans la première unité de séparation (1) ;
- la collecte d'une fraction enrichie en la substance cible dans la première cuve (4) ;
- l'inactivation virale de la fraction enrichie en la substance cible, ladite inactivation virale comprenant :
▪ le passage de la fraction enrichie en la substance cible collectée dans la première cuve (4) dans la deuxième unité de séparation (2), par la ligne de contournement (9) ;
▪ le passage d'une solution d'inactivation virale dans la deuxième unité de séparation (2), par la ligne de contournement (9) ;
▪ le mélange de la fraction enrichie en la substance cible et de la solution d'inactivation virale ; et
▪ la collecte dans la seconde cuve (5) du mélange de la fraction enrichie en la substance cible et de la solution d'inactivation virale ;
de manière à obtenir une fraction déplétée en virus actif ;
- le traitement de la fraction déplétée en virus actif par une étape de chromatographie dans la deuxième unité de séparation (2) ; et
- la collecte en sortie de la deuxième unité de séparation (2) d'une fraction plus enrichie en la substance cible.

2. Procédé selon la revendication 1, dans lequel le traitement du flux de fluide à traiter par une étape de chromatographie dans la première unité de séparation (1) est un traitement par chromatographie d'affinité et/ou le traitement de la fraction déplétée en virus actif par une étape de chromatographie dans la deuxième unité de séparation (2) est un traitement par chromatographie d'échange d'ions, de préférence un traitement par chromatographie d'échange d'anions.

3. Procédé selon la revendication 1 ou 2, dans lequel l'étape de passage de la fraction enrichie en la substance cible collectée dans la première cuve (4) dans la deuxième unité de séparation (2), par la ligne de contournement (9), et l'étape de passage d'une solution d'inactivation virale dans la deuxième unité de séparation (2), par la ligne de contournement (9), sont concomitantes, l'étape de mélange de la fraction enrichie en la substance cible et de la solution d'inactivation virale étant effectuée en ligne dans la ligne de contournement (9) de la deuxième unité de séparation (2) ; ou l'étape de passage de la fraction enrichie en la substance cible collectée dans la première cuve (4) dans la deuxième unité de séparation (2), par la ligne de contournement (9), et l'étape de passage d'une solution d'inactivation virale dans la deuxième unité de séparation (2), par la ligne de contournement (9), sont successives, l'étape de mélange de la fraction enrichie en la substance cible et de la solution d'inactivation virale étant effectuée dans la seconde cuve (5).

4. Procédé selon l'une des revendications 1 à 3, dans lequel le mélange de la fraction enrichie en la substance cible et de la solution d'inactivation virale collecté dans la seconde cuve (5) est stocké dans la seconde cuve (5) pendant au moins 15 min, de préférence pendant une durée allant de 20 à 45 min, de préférence encore sous agitation.

5. Procédé selon l'une des revendications 1 à 3, dans lequel l'inactivation virale de la fraction enrichie en la substance cible comprend en outre :
a) le passage du mélange de la fraction enrichie en la substance cible et de la solution d'inactivation virale collecté dans la seconde cuve (5) dans la deuxième unité de séparation (2), par la ligne de contournement (9) ;
b) la collecte du mélange en sortie de la deuxième unité de séparation (2) dans la seconde cuve (5) ;
les étapes a) et b) étant optionnellement répétées une ou plusieurs fois ou effectuées en continu ; et
c) optionnellement le stockage du mélange dans la seconde cuve (5), de préférence sous agitation ;
l'ensemble des étapes a), b), éventuellement répétées, ou éventuellement effectuées en continu, et de l'étape optionnelle c) étant de préférence effectuées pendant une durée d'au moins 15 min, plus préférentiellement pendant une durée de 20 à 45 min.

6. Procédé selon l'une des revendications 1 à 5, dans lequel la deuxième unité de séparation (2) comprend en outre un détecteur de pH (6) et/ou un détecteur de température (7), de préférence un détecteur en ligne, l'inactivation virale de la fraction enrichie en la substance cible comprenant en outre la mesure et, éventuellement le réajustement, du pH et/ou de la température du mélange de la fraction enrichie en la substance cible et de la solution d'inactivation virale passant dans la deuxième unité de séparation (2).

7. Procédé selon l'une des revendications 1 à 6, dans lequel la solution d'inactivation virale est une solution acide, le mélange de la fraction enrichie en la substance cible et de la solution acide ayant de préférence un pH inférieur ou égal à 5, de préférence encore inférieure ou égal à 4.

8. Procédé selon la revendication 7, comprenant, avant l'étape de traitement de la fraction déplétée en virus actif par une étape de chromatographie dans la deuxième unité de séparation (2), une étape d'ajout d'une solution basique dans la fraction déplétée en virus actif ; de préférence l'ajout de la solution basique dans la fraction déplétée en virus actif est effectué en ligne dans la deuxième unité de séparation (2) ou dans la seconde cuve (5), en passant de préférence la solution basique dans la deuxième unité de séparation (2), par la ligne de contournement (9).

9. Procédé selon l'une des revendications 1 à 8, comprenant au moins une étape de rinçage de la deuxième unité de séparation (2) dans laquelle un tampon de rinçage est passé dans la deuxième unité de séparation (2), par la ligne de contournement (9), de préférence après l'étape d'inactivation virale de la fraction enrichie en la substance cible et/ou avant l'étape de traitement de la fraction déplétée en virus actif par une étape de chromatographie dans la deuxième unité de séparation (2) et/ou après l'étape de traitement de la fraction déplétée en virus actif par une étape de chromatographie dans la deuxième unité de séparation (2).

10. Procédé selon l'une des revendications 1 à 9, dans lequel l'installation comprend en outre une troisième unité de séparation (3) comprenant au moins une colonne de chromatographie, une entrée de fluide et une sortie de fluide, dans laquelle une entrée de fluide est en connexion fluidique avec une sortie de fluide de la deuxième unité de séparation (2) ;
le procédé comprenant en outre les étapes suivantes :
- le traitement de la fraction plus enrichie en la substance cible par une étape de chromatographie dans la troisième unité de séparation (3), qui est de préférence un traitement par chromatographie d'échange d'ions, de préférence encore un traitement par chromatographie d'échange de cations ; et
- la collecte en sortie de la troisième unité de séparation (3) d'une fraction purifiée de la substance cible.

11. Procédé selon l'une des revendications 1 à 10, dans lequel le fluide à traiter est un surnageant de culture cellulaire et/ou la substance cible est un anticorps.

12. Procédé selon l'une des revendications 1 à 11, dans lequel la première unité de séparation (1) est une unité multicolonne.

13. Programme d'ordinateur comprenant des instructions de code de programme pour l'exécution des étapes du procédé selon l'une des revendications 1 à 12 lorsque ledit programme est exécuté sur un ordinateur.

14. Support de stockage lisible par ordinateur sur lequel est enregistré un programme d'ordinateur selon la revendication 13.

15. Système comprenant un processeur couplé à une mémoire sur laquelle est enregistré un programme d'ordinateur selon la revendication 13.

## Patentansprüche

1. Verfahren zur Reinigung einer Zielsubstanz aus einem zu behandelnden Fluid, die mindestens eine Verunreinigung umfasst, in einer Anlage, die umfasst:
- eine erste Trenneinheit (1), die mindestens eine Chromatographiesäule, einen Fluideinlass und einen Fluidauslass umfasst;
- eine zweite Trenneinheit (2), die mindestens eine Chromatographiesäule, einen Fluideinlass (10), einen Fluidauslass (11) und eine Bypassleitung (9) umfasst, die mit mindestens einem Fluideinlasss (10) der zweiten Trenneinheit an einem ihrer Enden und mit mindestens einem Fluidauslass (11) der zweiten Trenneinheit am anderen ihrer Enden verbunden ist und die Chromatographiesäule/n der zweiten Trenneinheit (2) umgeht;
- einen ersten Tank (4) in Fluidverbindung mit einem Fluidauslass der ersten Trenneinheit (1) und mit einem Fluideinlass (10) der zweiten Trenneinheit (2); und
- einen zweiten Tank (5) in Fluidverbindung mit mindestens einem Fluideinlass (10) und einem Fluidauslass (11) der zweiten Trenneinheit (2);
wobei das Verfahren die folgenden Schritte umfasst:
- das Bereitstellen eines Stroms des zu behandelnden Fluids;
- das Behandeln des zu behandelnden Fluidstroms durch einen Chromatographieschritt in der ersten Trenneinheit (1);
- das Sammeln einer mit der Zielsubstanz angereicherten Fraktion im ersten Tank (4);
- die virale Inaktivierung der mit der Zielsubstanz angereicherten Fraktion, wobei die virale Inaktivierung umfasst:
▪ das Leiten der im ersten Tank (4) gesammelten, mit der Zielsubstanz angereicherten Fraktion durch die Bypassleitung (9) in die zweite Trenneinheit (2);
▪ das Leiten einer Virusinaktivierungslösung durch die Bypassleitung (9) in die zweite Trenneinheit (2);
▪ das Mischen der mit der Zielsubstanz angereicherten Fraktion und der Virusinaktivierungslösung; und
▪ das Sammeln des Gemischs aus der mit der Zielsubstanz angereicherten Fraktion und der Virusinaktivierungslösung im zweiten Tank (5);
derart, dass eine von aktiven Viren befreite Fraktion erhalten wird;
- das Behandeln der von aktiven Viren befreiten Fraktion durch einen Chromatographieschritt in der zweiten Trenneinheit (2); und
- das Sammeln einer stärker mit der Zielsubstanz angereicherten Fraktion am Ausgang der zweiten Trenneinheit (2).

2. Verfahren nach Anspruch 1, wobei die Behandlung des zu behandelnden Fluidstroms durch einen Chromatographieschritt in der ersten Trenneinheit (1) eine Behandlung durch Affinitätschromatographie und/oder die Behandlung der von aktiven Viren befreiten Fraktion durch einen Chromatographieschritt in der zweiten Trenneinheit (2) eine Behandlung durch Ionenaustauschchromatographie, vorzugsweise eine Behandlung durch Anionenaustauschchromatographie, ist.

3. Verfahren nach Anspruch 1 oder 2, wobei der Schritt des Leitens der im ersten Tank (4) gesammelten, mit der Zielsubstanz angereicherten Fraktion durch die Bypassleitung (9) in die zweite Trenneinheit (2) und der Schritt des Leitens einer Virusinaktivierungslösung durch die Bypassleitung (9) in die zweite Trenneinheit (2) gleichzeitig erfolgen, wobei der Schritt des Mischens der mit der Zielsubstanz angereicherten Fraktion und der Virusinaktivierungslösung inline in der Bypassleitung (9) der zweiten Trenneinheit (2) erfolgt; oder wobei der Schritt des Leitens der im ersten Tank (4) gesammelten, mit der Zielsubstanz angereicherten Fraktion durch die Bypassleitung (9) in die zweite Trenneinheit (2) und der Schritt des Leitens einer Virusinaktivierungslösung durch die Bypassleitung (9) in die zweite Trenneinheit (2) nacheinander erfolgen, wobei der Schritt des Mischens der mit der Zielsubstanz angereicherten Fraktion und der Virusinaktivierungslösung im zweiten Tank (5) durchgeführt wird.

4. Verfahren nach einem der Ansprüche 1 bis 3, wobei das im zweiten Tank (5) gesammelte Gemisch aus der mit der Zielsubstanz angereicherten Fraktion und der Virusinaktivierungslösung im zweiten Tank (5) mindestens 15 Minuten, vorzugsweise 20 bis 45 Minuten, vorzugsweise weiterhin unter Rühren, gelagert wird.

5. Verfahren nach einem der Ansprüche 1 bis 3, wobei die Virusinaktivierung der mit der Zielsubstanz angereicherten Fraktion ferner umfasst:
a) das Leiten des Gemisches aus der mit der Zielsubstanz angereicherten Fraktion und der in dem zweiten Tank (5) gesammelten Virusinaktivierungslösung durch die Bypassleitung (9) in die zweite Trenneinheit (2);
b) das Sammeln des Gemisches am Ausgang der zweiten Trenneinheit (2) im zweiten Tank (5);
wobei die Schritte a) und b) optional ein- oder mehrmals wiederholt oder kontinuierlich durchgeführt werden; und
c) optional das Lagern des Gemischs im zweiten Tank (5), vorzugsweise unter Rühren;
wobei die Gesamtheit der gegebenenfalls wiederholten oder gegebenenfalls kontinuierlich durchgeführten Schritte a), b) und des optionalen Schritts c) vorzugsweise über einen Zeitraum von mindestens 15 Minuten, noch bevorzugter über einen Zeitraum von 20 bis 45 Minuten, durchgeführt wird.

6. Verfahren nach einem der Ansprüche 1 bis 5, wobei die zweite Trenneinheit (2) ferner einen pH-Sensor (6) und/oder einen Temperatursensor (7), vorzugsweise einen Inline-Sensor, umfasst, wobei die Virusinaktivierung der mit der Zielsubstanz angereicherten Fraktion ferner die Messung und gegebenenfalls die Nachjustierung des pH-Werts und/oder der Temperatur des Gemischs aus der mit der Zielsubstanz angereicherten Fraktion und der Virusinaktivierungslösung umfasst, die in die zweite Trenneinheit (2) fließt.

7. Verfahren nach einem der Ansprüche 1 bis 6, wobei die Virusinaktivierungslösung eine saure Lösung ist, wobei das Gemisch aus der mit der Zielsubstanz angereicherten Fraktion und der sauren Lösung einen pH-Wert von vorzugsweise unter oder gleich 5, vorzugsweise ebenfalls von unter oder gleich 4, hat.

8. Verfahren nach Anspruch 7, umfassend vor dem Schritt der Behandlung der von aktiven Viren befreiten Fraktion durch einen Chromatographieschritt in der zweiten Trenneinheit (2) einen Schritt des Hinzufügens einer basischen Lösung zu der von aktiven Viren befreiten Fraktion; wobei vorzugsweise das Hinzufügen der basischen Lösung zu der von aktiven Viren befreiten Fraktion inline in der zweiten Trenneinheit (2) oder im zweiten Tank (5) erfolgt, wobei die basische Lösung vorzugsweise durch die Bypassleitung (9) in die zweite Trenneinheit (2) fließt.

9. Verfahren nach einem der Ansprüche 1 bis 8, umfassend mindestens einen Spülschritt der zweiten Trenneinheit (2), bei dem ein Spülpuffer durch die Bypassleitung (9) in die zweite Trenneinheit (2) geleitet wird, vorzugsweise nach dem Schritt der Virusinaktivierung der mit der Zielsubstanz angereicherten Fraktion und/oder vor dem Schritt der Behandlung der von aktiven Viren befreiten Fraktion durch einen Chromatographieschritt in der zweiten Trenneinheit (2) und/oder nach dem Schritt der Behandlung der von aktiven Viren befreiten Fraktion durch einen Chromatographieschritt in der zweiten Trenneinheit (2).

10. Verfahren nach einem der Ansprüche 1 bis 9, wobei die Anlage ferner eine dritte Trenneinheit (3) umfasst, die mindestens eine Chromatographiesäule, einen Fluideinlass und einen Fluidauslass umfasst, wobei ein Fluideinlass in Fluidverbindung mit einem Fluidauslass der zweiten Trenneinheit (2) steht;
wobei das Verfahren ferner die folgenden Schritte umfasst:
- die Behandlung der mit der Zielsubstanz stärker angereicherten Fraktion durch einen Chromatographieschritt in der dritten Trenneinheit (3), die vorzugsweise eine Behandlung durch Ionenaustauschchromatographie, vorzugsweise weiterhin eine Behandlung durch Kationenaustauschchromatographie, ist; und
- das Sammeln einer gereinigten Fraktion der Zielsubstanz am Ausgang der dritten Trenneinheit (3).

11. Verfahren nach einem der Ansprüche 1 bis 10, wobei das zu behandelnde Fluid ein Zellkulturüberstand und/oder die Zielsubstanz ein Antikörper ist.

12. Verfahren nach einem der Ansprüche 1 bis 11, wobei die erste Trenneinheit (1) eine Mehrsäuleneinheit ist.

13. Rechnerprogramm, das Programmcodeanweisungen zur Ausführung der Schritte des Verfahrens nach einem der Ansprüche 1 bis 12 umfasst, wenn das Programm auf einem Rechner ausgeführt wird.

14. Rechnerlesbares Speichermedium, auf dem ein Rechnerprogramm nach Anspruch 13 gespeichert ist.

15. System, das einen Prozessor umfasst, der mit einem Speicher gekoppelt ist, auf dem ein Rechnerprogramm nach Anspruch 13 gespeichert ist.

## Claims

1. A method for purifying a target substance from a fluid to be treated comprising at least one impurity, in an installation comprising:
- a first separation unit (1) comprising at least a chromatography column, a fluid inlet and a fluid outlet;
- a second separation unit (2) comprising at least a chromatography column, a fluid inlet (10), a fluid outlet (11) and a bypass line (9) linked to at least a fluid inlet (10) of the second separation unit at one of its ends and to at least a fluid outlet (11) of the second separation unit at the other of its ends and bypassing the at least one chromatography column of the second separation unit (2);
- a first reservoir (4) in fluid connection with a fluid outlet of the first separation unit (1) and a fluid inlet (10) of the second separation unit (2); and
- a second reservoir (5) in fluid connection with at least a fluid inlet (10) and a fluid outlet (11) of the second separation unit (2);
the method comprising the following steps:
- providing a flow of fluid to be treated;
- treating the flow of fluid to be treated by means of a chromatography step in the first separation unit (1);
- collecting a fraction enriched in the target substance in the first reservoir (4);
- the viral inactivation of the fraction enriched in the target substance, said viral inactivation comprising:
▪ passing the fraction enriched in the target substance collected in the first reservoir (4) through the second separation unit (2), via the bypass line (9);
▪ passing a viral inactivation solution through the second separation unit (2), via the bypass line (9);
▪ mixing the fraction enriched in the target substance and the viral inactivation solution; and
▪ collecting the mixture of the fraction enriched in the target substance and the viral inactivation solution in the second reservoir (5);
so as to obtain a fraction depleted of active virus;
- treating the fraction depleted of active virus by means of a chromatography step in the second separation unit (2); and
- collecting a fraction more enriched in the target substance at the outlet of the second separation unit (2).

2. The method according to claim 1, wherein the treatment of the flow of fluid to be treated by means of a chromatography step in the first separation unit (1) is an affinity chromatography treatment and/or the treatment of the fraction depleted of active virus by means of a chromatography step in the second separation unit (2) is a treatment by ion exchange chromatography, preferably a treatment by anion exchange chromatography.

3. The method according to claim 1 or 2, wherein the step of passing the fraction enriched in the target substance collected in the first reservoir (4) through the second separation unit (2), via the bypass line (9), and the step of passing a viral inactivation solution through the second separation unit (2), via the bypass line (9), are simultaneous, the step of mixing the fraction enriched in the target substance and the viral inactivation solution being carried out online in the bypass line (9) of the second separation unit (2); or the step of passing the fraction enriched in the target substance collected in the first reservoir (4) through the second separation unit (2), via the bypass line (9), and the step of passing a viral inactivation solution through the second separation unit (2), via the bypass line (9), are successive, the step of mixing the fraction enriched in the target substance and the viral inactivation solution being carried out in the second reservoir (5).

4. The method according to any of claims 1 to 3, wherein the mixture of the fraction enriched in the target substance and the viral inactivation solution collected in the second reservoir (5) is stored in the second reservoir (5) for at least 15 min, preferably for a duration from 20 to 45 min, still preferably under stirring.

5. The method according to any of claims 1 to 3, wherein the viral inactivation of the fraction enriched in the target substance further comprises:
a) passing the mixture of fraction enriched in the target substance and viral inactivation solution collected in the second reservoir (5) through the second separation unit (2) via the bypass line (9);
b) collecting the mixture at the outlet of the second separation unit (2) in the second reservoir (5);
steps a) and b) are optionally repeated one or more times or carried out continuously; and
c) optionally storing the mixture in the second reservoir (5); preferably under stirring;
the steps a) and b), which are optionally repeated or optionally carried out continuously, and the optional step c) taken together being carried out for a duration of at least 15 min, more preferably for a duration from 20 to 45 min.

6. The method according to any of claims 1 to 5, wherein the second separation unit (2) further comprises a pH sensor (6) and/or a temperature sensor (7), preferably an online sensor, the viral inactivation of the fraction enriched in the target substance further comprising measuring and optionally adjusting the pH and/or temperature of the mixture of the fraction enriched in the target substance and the viral inactivation solution passing through the second separation unit (2).

7. The method according to any of claims 1 to 6, wherein the viral inactivation solution is an acidic solution, the mixture of the fraction enriched in the target substance and the acidic solution having preferably a pH less than or equal to 5, still preferably less than or equal to 4.

8. The method according to claim 7, comprising a step of adding a basic solution to the fraction depleted of active virus before the step of treating the fraction depleted of active virus by means of a chromatography step in the second separation unit (2), preferably the addition of the basic solution to the fraction depleted of active virus is carried out online in the second separation unit (2) or is carried out in the second reservoir (5), by passing preferably the basic solution in the second separation unit (2), via the bypass line (9).

9. The method according to any of claims 1 to 8, comprising at least one step of rinsing the second separation unit (2) wherein a rinse buffer is passed through the second separation unit (2), via the bypass line (9), preferably after the step of viral inactivation of the fraction enriched in the target substance and/or before the step of treating the fraction depleted of active virus by means of a chromatography step in the second separation unit (2) and/or after the step of treating the fraction depleted of active virus by means of a chromatography step in the second separation unit (2).

10. The method according to any of claims 1 to 9, wherein the installation further comprises a third separation unit (3) comprising at least a chromatography column, a fluid inlet and a fluid outlet, wherein a fluid inlet is in fluid connection with a fluid outlet of the second separation unit (2);
the method further comprising the following steps:
- treating the fraction more enriched in the target substance by means of a chromatography step in the third separation unit (3), which is preferably a treatment by ion exchange chromatography, still preferably a treatment by cation exchange chromatography; and
- collecting a purified fraction of the target substance at the outlet of the third separation unit (3).

11. The method according to any of claims 1 to 10, wherein the fluid to be treated is a cell culture supernatant and/or the target substance is an antibody.

12. The method according to any of claims 1 to 11, wherein the first separation unit (1) is a multi-column unit.

13. A computer program comprising program code instructions for the execution of the steps of the method according to any of claims 1 to 12 when said program is run on a computer.

14. A computer-readable storage medium on which a computer program according to claim 13 is stored.

15. A system comprising a processor coupled to a memory on which a computer program according to claim 13 is stored.
